# EUROPEAN PATENT APPLICATION

(11) **EP 3 078 668 A1**
(43) Date of publication of application: **12.10.2016**
(21) Application number: 15162892.2
(22) Date of filing: 09.04.2015
(51) Int. Cl.: C07F 9/34, H01B 1/12, C01B 25/10, C07F 9/535, C07C 211/63

(54) **METHOD FOR PREPARATION OF FLUORO CYANO COMPOUNDS OF THE 15TH GROUP WITH A BRONSTEDT ACID**

(71) Applicant: Lonza Ltd, 3930 Visp (CH)
(72) Inventor: Ellinger, Stefan, 3930 Visp (CH); Täschler, Christoph, 3930 Visp (CH); Zur Täschler, Cornelia, 3930 Visp (CH); Sievert, Katharina, 18055 Rostock (DE); Schulz, Axel, 18059 Rostock (DE); Harloff, Jörg, 18147 Rostock (DE)

(57) **Abstract**

The invention discloses a method for preparation of fluoro cyano compounds of the 15th group of the periodic table with 1 to 5 cyano residues, represented by formula (I),

[Catⁿ⁺][(Z¹F₆₋ₘ(CN)ₘ)⁻]ₙ (I)

by a reaction of compound of formula (A1) with trimethylsilylcyanide in the presence of a Bronstedt acid;

[Catⁿ⁺][(Z¹F₆)⁻]ₙ (A1)

Catⁿ⁺ is a cation, Z¹ is P, As, Sb or Bi, m is an integer from 1 to 5 and n is 1, 2, 3 or 4.
Preferred compounds prepared by the claimed method are formulae (1)-(4b)

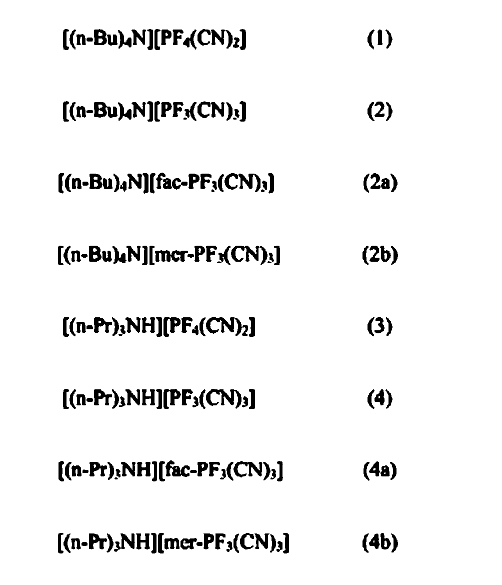

## Description

The invention discloses a method for preparation of fluoro cyano compounds of the 15th group of the periodic table with 1 to 5 cyano residues, represented by formula (I),

[Catⁿ⁺][(Z¹P₆₋ₘ(CN)ₘ)⁻]ₙ (I)

by a reaction of compound of formula (A1) with trimethylsilylcyanide in the presence of a Bronstedt acid;

[Catⁿ⁺][(Z¹F₆)⁻]ₙ (A1)

Catⁿ⁺ is a cation, Z¹ is P, As, Sb or Bi, m is an integer from 1 to 5 and n is 1, 2, 3 or 4.

### BACKGROUND OF THE INVENTION

The term "ionic liquid" (IL) is usually used to refer to a salt which is liquid at temperatures below 100°C, in particular at room temperature. Such liquid salts typically comprise organic cations and organic or inorganic anions, and are described inter alia in P. Wasserscheid et al., Angew. Chem., 2000, 112, 3926-3945.

Ionic liquids have a series of interesting properties: Usually, they are thermally stable, relatively non-flammable and have a low vapor pressure. They show good solvability for numerous organic and inorganic substances. In addition, ionic liquids have interesting electrochemical properties, for example electrical conductivity which is often accompanied by a high electrochemical stability.

These attributes give rise to many applications of ionic liquids: They can be used for example as solvent in synthesis, as electrolyte, as lubricant and as hydraulic fluid. Moreover they serve as phase-transfer catalyst, as extraction medium, as heat-transfer medium, as surface-active substance, as plasticizer, as conductive salt, organic salt or additive in electrochemical cells, as electrolyte, as component in electrolyte formulations, wherein such electrolyte formulation comprising an ionic liquid is preferably used in electrochemical and/or optoelectronic device such as a photovoltaic cell, a light emitting device, an electrochromic or photo-electrochromic device, an electrochemical sensor and/or biosensor, particularly preferred in a dye sensitized solar cell.

Therefore, there is a fundamental need for ionic liquids having a variety of properties which open up additional opportunities for their use.

K. B. Dillon et al., J. Chem. Soc., Chem. Commun., 1983, 1089-1090, mentions a trans-PF₂(CN)₄ anion, but the cation is not identified.

EP 2 410 601 A1 discloses ionic liquids comprising a cyan phosphate-based anion represented by the formula P(CN)ₙX₆₋ₙ with X being halogen. Special emphasis is given to certain specific imidazolium salts of P(CN)₃X₃ with X being F or Cl, since all exemplified substances have n = 3 and are trichloro or tri flouro tricyanophosphates. The preparation starts with imidazolium chloride and PCl₅, then reaction with AgCN providing the respective imidazolium P(CN)₃Cl₃. In order to obtain the respective imidazolium P(CN)₃F₃, a third step is necessary, that is reaction with AgBF₄ as fluorinating agent. In example 4 even a fourth step follows, addition of imidazolium chloride.

US 2013/0089777 A1 discloses a material for use as an electrolyte comprising a lithium salt which comprises the following components (A1) and (B), or which comprises the following components (A1), (A2) and (B):
(A1) a lithium cation;
(A2) an organic cation;
(B) a cyanofluorophosphate anion represented by the following general formula (J1):

   ⁻P(CN)ₙF₆₋ₙ (J1)

   wherein n is an integer of 1 to 5.

Li P(CN)₃F₃ is the only compound disclosed as substance. Its preparation starts with AgCN and PCl₃ providing P(CN)₃, which is converted with LiCl and gaseous Cl₂ to Li P(CN)₃(Cl)₃, which is converted with AgBF₄ to Ag P(CN)₃(F)₃, which is converted with LiI to the desired Li P(CN)₃(Cl)₃.

The only two electrolytes disclosed comprise a Li⁺ cation, a trifluorotricyanophosphate anion and optionally a 1-ethyl-3-methylimidazolium cation.

JP 2012 009158 A discloses an electrolyte useful in a lithium secondary battery comprising components (A1), (A2) and (B), with (A1) being a Lithium cation, (A2) being an organic cation and (B) being a cyanofluorophosphate-based anion represented by the general formula P(CN)ₙF₆₋ₙ, wherein n is an integer of 1 to 5.

1-ethyl-3-methylimidazolium trifluorotricyanophosphate is the only compound disclosed as substance. Its preparation is identical with the preparation disclosed in EP 2 410 601 A1 and starts with PCl₅ and 1-ethyl-3-methylimidazolium chloride, then reaction with AgCN providing 1-ethyl-3-methylimidazolium P(CN)₃Cl₃. In order to obtain the respective imidazolium P(CN)₃F₃, a third step is necessary, that is reaction with AgBF₄ as fluorinating agent, then a fourth step follows, addition of 1-ethyl-3-methylimidazolium chloride. The only one specific electrolyte disclosed contains a Li⁺ cation, a 1-ethyl-3-methylimidazolium cation and a trifluorotricyanophosphate anion.

JP 2012 248515 A discloses a metal salt useful for an electrode protective film forming agent, the metal salt comprises a component (A1) or a component (A2), and a component (B), (A1) being a monovalent metal cation (excluding a lithium cation), (A2) being a divalent metal cation and (B) being a cyanofluorophosphate type anion represented by the formula ⁻P(CN)ₙF₆₋ₙ, wherein n is an integer of 1 to 5.

Only one embodiment is exemplified, which is Ag P(CN)₃F₃. Its preparation is identical with the initial preparation steps disclosed in US 2013/0089777 A1 and starts with AgCN and PCl₃ providing P(CN)₃, which is converted with LiCl and gaseous Cl₂ to Li P(CN)₃(Cl)₃, which is converted with AgBF₄ to Ag P(CN)₃(F)₃. No example is disclosed for the embodiment component (A2), which is a divalent metal cation.

There was a need for a simplified method with high yield and satisfactory purity for the preparation of fluoro cyanide compounds of the 15th group of the periodic table with the anion having the general formula [(Z¹F₆₋ₘ(CN)ₘ)⁻] with Z¹ being P, As, Sb or Bi and m being 1, 2, 3, 4 or 5. The method should require as few steps as possible. The method should allow also the preparation of compounds with m being 1, 2, 4 or 5 and not only of compound with m being 3. The method should avoid the use of Cl₂, AgCN or AgBF₄. The method should provide stable compounds of said formula which can be used as ionic liquids or as precursors of ionic liquids and can be used e.g. in electrolyte formulations and in electrochemical or optoelectronic devices. These compounds should be able to be disposed of in an environmentally friendly manner after use.

The method should allow the preparation of the desired compounds in high yields and under mild conditions with respect to methods disclosed in the prior art.

This object is achieved by a method using trimethylsilylcyanide as CN source and by doing the reaction in the presence of a Bronstedt acid. No Cl₂, AgCN or AgBF₄ is required. Another advantage is that the reaction does not require mandatorily an extra solvent. The method has a reduced number of steps compared to the methods known from the prior art. In particular the method allows to start already with a salt of a hexafluoro anion of an element the 15th group of the periodic table having as cation the desired cation of the final product. Thereby the additional step of a metathesis reaction is no longer required. This is an advantage compared to the methods of prior art mentioned above, where always either AgBF₄ or AgCN is needed and where in some cases even an additional step of metathesis is needed to provide the desired various salts with cations other than Li⁺ or Ag⁺. The method allows for the preparation not only of compounds with m = 3, but also for compounds with m = 1, 2, 4 or 5, and these compounds can be prepared specifically and individually, and not only as mixtures. The conditions of the reaction are milder than those disclosed in the methods of the prior art, the reaction can be done at lower temperature or in shorter time.

In this text, the following meanings are used, if not otherwise stated:
- alkyl: linear or branched alkyl;
- C_{1-q} alkyl: refers to any alkyl residue which contains from 1 to q carbon atoms; for example C₁₋₆ alkyl encompasses inter alia methyl, ethyl, propyl, isopropyl, n-butyl, isobutyl, sec-butyl, tert-butyl, n-pentyl, isopentyl (3-methylbutyl), neopentyl (2,2-dimethylpropyl), n-hexyl and isohexyl (4-methylpentyl);
- C_{2-q} alkenyl: refers to an alkenyl residue which contains from 2 to q carbon atoms and contains at least one double bond, the carbon chain can be linear or branched; for example C₂₋₄ alkenyl encompasses inter alia ethenyl, 1-methylethenyl, prop-1-enyl, prop-2-enyl, 2-methylprop-2-enyl and buta-1,3-dienyl;
- C_{2-q} alkynyl: refers to an alkynyl residue which contains from 2 to q carbon atoms and contains at least one triple bond, the carbon chain can be linear or branched; for example C₂₋₄ alkynyl encompasses inter alia ethynyl, prop-1-ynyl and prop-2-ynyl;
- C₆₋₁₀ aryl: refers to an aryl residue which has from 6 to 10 carbon atoms and is unsubstituted or substituted by 1, 2, 3 or 4 identical or different substituents independently from each other selected from the group consisting of C₁₋₄ alkyl and C₁₋₄ alkoxy; for example C₆₋₁₀ aryl encompasses inter alia phenyl, methylphenyl, methoxyphenyl, dimethylphenyl, ethylmethylphenyl, diethylphenyl and naphthyl;
- cyclic alkyl or cycloalkyl: include cyclo and polycyclo, such as bicyclo or tricyclo, aliphatic residues;
- C_{3-q} cycloalkyl: refers to a cycloalkyl group having from 3 to q carbon atoms; for example C₃₋₁₀ cycloalkyl encompasses inter alia cyclopropyl, cyclobutyl, cyclopentyl, cyclohexyl, cycloheptyl, cyclooctyl, cyclononyl and cyclodecyl;

- C_{1-q} alkoxy: refers to an linear or branched alkoxy group having from 1 to q carbon atoms; for example C₁₋₂₀ alkoxy encompasses inter alia methoxy, ethoxy, propoxy, isopropoxy, n-butoxy, isobutoxy, sec-butoxy, tert-butoxy, pentyloxy, 1,4-dimethylpentyloxy, hexyloxy, heptyloxy, octyloxy, 1,5-dimethylhexyloxy, nonyloxy, decyloxy, 4-ethyl-1,5-dimethylhexyloxy, undecyloxy, dodecyloxy, tridecyloxy, tetradecyloxy and eicosyloxy;
- alkylene: means a linear or branched alkylene group; e.g. propylene, and e.g. propylene can be connected via its C1 and C2 carbon atoms (a branched alkylene group), or via its C1 and C3 carbon atoms (linear alkylene group);
- DCM: dichloromethane;
- EMIm: 1-ethyl-3-methylimidazolium 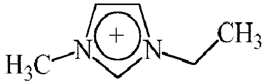
- eq.: molar equivalent;
- halide: F⁻ , Cl⁻, Br⁻ or I⁻ , preferably F⁻ , Cl⁻ or Br⁻ , more preferably Cl⁻;
- halogen: F, Cl, Br or I; preferably F, Cl or Br;
- IL: ionic liquid;
- "linear" and "n-": are used synonymously with respect to the respective isomers of alkanes;
- RT: room temperature, it is used synonymously with the expression ambient temperature;
- T_{dec}: decomposition temperature;
- THF: tetrahydrofuran;
- TMSCN: (CH₃)₃SiCN, i.e. trimethylsilylcyanide;
- triflic acid: trifluoromethanesulfonic acid, CF₃SO₃H;
- Trityl: means the trityl cation, i.e. [Ph₃C]⁺;
"wt%", "% by weight" and "weight-%" are used synonymously and mean percent by weight.

The expressions dye sensitized solar cell and photosensitized solar cell are used synonymously.

### SUMMARY OF THE INVENTION

Subject of the invention is a method for the preparation of compound of formula (I);

[Catⁿ⁺][(Z¹F₆₋ₘ(CN)ₘ)⁻]ₙ (I)

the method comprises a step STEP1;
STEP1 comprises a reaction REAC1, wherein compound of formula (A1)

[Catⁿ⁺][(Z¹F₆)⁻]ₙ (A1)

is reacted with trimethylsilylcyanide in the presence of a compound CATACID;
- CATACID: is selected from the group consisting of HF, HCl, HBr, HI, HNO₃, H₂SO₃, H₂SO₄, NaHSO₄, Oleum, KHSO₄, NaHCO₃, KHCO₃, H₃PO₄, H₃PO₃, HSO₃F, HSO₃Cl, CH₃COOH, CF₃COOH, C(Cl₃)COOH, methansulfonic acid, HCOOH, AR-COOH, AR-SO₃H, C(OH)(COOH)₃, CF₃SO₃H, HCN, HOCN, HSCN, HSeCN, HNCO, HNCS, HN₃, HQ20(X1)4, polymolybdate, polytungstate, HQ21(X1)₆, and mixtures thereof;
- AR: is Phe or Phe substituted with 1, 2 or 3 identical or different residues selected from the group consisting of halogen and CH₃;
- Q20: is B, Al, Ga, In or Th;
- Q21: is P, As, Sb or Bi;
- X1: is halogen;
- Z¹: is selected from the group consisting of P, As, Sb and Bi;
- m: is 1, 2, 3, 4 or 5;
- n: is 1, 2, 3 or 4;
- Catⁿ⁺: is selected from the group consisting of inorganic cation CatINORGⁿ⁺ and organic cation CatORGⁿ⁺,
- CatINORGⁿ⁺: is a cation selected from the 1., 2., 3., 4., 5., 6., 7., 8., 9., 10., 11., 12., 13., 14., 15. or 16. group of the periodic table, or is a cation from the lanthanides or is a cation from the actinides or is NH₄⁺;
- CatORGⁿ⁺: is selected from the group consisting of CatORG-A⁺, CatORG-B⁺, CatORG-C⁺, [(CH₃)₃SiFSi(CH₃)₃]⁺, Ph₃C⁺, guanidinium and (H₂(R18)N-R16-N(R19)H₂)²⁺;
- CatORG-A⁺: is (WR2R3R4R5)⁺,
wherein
- W: is a nitrogen or phosphorus; and

(i)
   - R2, R3, R4 and R5: are identical or different and independently from each other selected from the group consisting of H, C₁₋₂₀ alkyl, C₁₋₂₀ perfluoroalkyl, C₃₋₁₀ cycloalkyl and C₆₋₁₀ aryl, with the proviso, that at least one of the residues R2, R3, R4 and R5 is not H; or
(ii)
   - R2 and R3: together are a hydrocarbon chain and form together with W a 5- to 7-membered saturated or unsaturated heterocyclic ring,
   - R4 and R5: are identical or different and independently from each other selected from the group consisting of H, C₁₋₂₀ alkyl, C₁₋₂₀ perfluoroalkyl, C₃₋₁₀ cycloalkyl and C₆₋₁₀ aryl; or
(iii)
   - R2 and R3: together are a hydrocarbon chain and form together with W, and R4 and R5 together are a hydrocarbon chain and form together with W, independently from each other, 5- to 7-membered saturated or unsaturated heterocyclic rings;
CatORG-B⁺ is (XR6R7R8)⁺,
wherein
- X: is nitrogen,
- R6 and R7: together are a hydrocarbon chain and form together with X a 5- to 7-membered unsaturated heterocyclic ring in which X is connected by a single bond and a double bond to R6 and R7 respectively,
- R8: is selected from the group consisting of H, C₁₋₂₀ alkyl, C₂₋₈ alkenyl, C₁₋₂₀ perfluoroalkyl, C₃₋₁₀ cycloalkyl or C₆₋₁₀ aryl;
CatORG-C⁺ is (YR9R10R11)⁺,
wherein
- Y: is sulphur;

(i)
   - R9, R10 and R11: are identical or different and independently from each other selected from the group consisting of H, C₁₋₂₀ alkyl, C₁₋₂₀ perfluoroalkyl, C₃₋₁₀ cycloalkyl and C₆₋₁₀ aryl; or
(ii)
   - R9 and R10: together are a hydrocarbon chain and form together with Y a 5- to 7-membered saturated or unsaturated ring,
   R11 is selected from the group consisting of H, C₁₋₂₀ alkyl, C₁₋₂₀ perfluoroalkyl, C₃₋₁₀ cycloalkyl and C₆₋₁₀ aryl;
the residues R2, R3, R4, R5, R6, R7, R8, R9, R10 and R11 are, independently from each other, unsubstituted or, where applicable, substituted by 1, 2, 3, 4, 5 or 6 substituents selected from the group consisting of C₁₋₄ alkyl, C₃₋₁₀ cycloalkyl, C₂₋₈ alkenyl, phenyl, benzyl, halogen, cyano and C₁₋₄ alkoxy;
in any of said hydrocarbon chains formed by R2 and R3, by R4 and R5, by R6 and R7, and by R9 and R10, 1 or 2 carbon atoms of said hydrocarbon chains can be exchanged for 1 or 2 heteroatoms respectively, said one or two heteroatoms being selected from the group consisting of O, N and S; in case of an exchange for N, this N is unsubstituted or substituted by a residue selected from the group consisting of C₁₋₈ alkyl, C₃₋₁₀ cycloalkyl, C₂₋₈ alkenyl and C₁₋₈ perfluoroalkyl;
- R16: is selected from the group consisting of C₂₋₈ alkylen, C₃₋₈ cycloalkylen, phenylen, C(H)(phenyl), R17(-O-R17)ₙ₁;
- R17: is selected from the group consisting of CH₂-CH₂, CH₂-CH₂-CH₂, CH₂-C(H)(CH₃)-CH₂, CH₂-CH₂-C(H)(CH₃) and CH₂-CH₂-CH₂-CH₂;
- R18 and R19: are identical or different and independently from each other selected from the group consisting of H, C₁₋₈ alkyl, C₃₋₈ cycloalkyl, phenyl and benzyl;
- n1: is an integer from 1 to 20.

### DETAILED DESCRIPTION OF THE INVENTION

Preferably, Z¹ is P, also in connection with any of the embodiments disclosed in the specification.

Preferably, m is 2, 3, 4 or 5;
more preferably, m is 3, 4 or 5;
even more preferably, m is 3 or 4;
also in connection with any of the embodiments disclosed in the specification.

Preferably, n is 1 or 2, also in connection with any of the embodiments disclosed in the specification.

Preferably, CATACID is selected from the group consisting of HF, HCl, HBr, H₂SO₄, NaHSO₄, Oleum, KHSO₄, H₃PO₄, HSO₃F, HSO₃Cl, CH₃COOH, CF₃COOH, C(Cl₃)COOH, methansulfonic acid, HCOOH, AR-SO₃H, C(OH)(COOH)₃, CF₃SO₃H, HCN, HQ20(X1)4, polymolybdate, polytungstate, HQ21(X1)₆, and mixtures thereof;
- AR: is Phe or Phe substituted with 1, 2 or 3 identical or different residues selected from the group consisting of halogen and CH₃;
- Q20: is B, Al or Ga;
- Q21: is P, Sb or Bi;
- X1: is F, Cl or Br.

More preferably, CATACID is selected from the group consisting of HF, HCl, HBr, H₂SO₄, Oleum, H₃PO₄, HSO₃F, HSO₃Cl, CH₃COOH, CF₃COOH, methansulfonic acid, AR-SO₃H, CF₃SO₃H, HCN, HQ20(X1)4, polymolybdate, polytungstate, and mixtures thereof;
- AR: is Phe substituted with 1 residue selected from the group consisting of F, Cl and CH₃;
- Q20: is B, Al or Ga;
- X1: is F, Cl or Br.

Even more preferably, CATACID is selected from the group consisting of HF, HCl, H₂SO₄, Oleum, H₃PO₄, HSO₃F, HSO₃Cl, CH₃COOH, CF₃COOH, methansulfonic acid, AR-SO₃H, CF₃SO₃H, HCN, HQ20(X1)4, and mixtures thereof;
- AR: is Phe substituted with 1 residue selected from the group consisting of F, Cl and CH₃;
- Q20: is B, Al or Ga;
- X1: isForCl.

Especially, CATACID is selected from the group consisting of HF, HCl, H₂SO₄, Oleum, HSO₃F, HSO₃Cl, CF₃COOH, methansulfonic acid, AR-SO₃H, CF₃SO₃H, and mixtures thereof;
- AR: is Phe substituted with CH₃.

More especially, CATACID is CF₃SO₃H.

When m is 4, then anion [(Z¹F₆₋ₘ(CN)ₘ)⁻] in formula (1) is preferably [(cis-PF₂(CN)₄)⁻].

When m is 3, then anion [(Z¹F₆₋ₘ(CN)ₘ)⁻] in formula (1) is preferably [(mer-PF₃(CN)₃)⁻].

Preferably, CatINORGⁿ⁺ is a cation selected from the 1., 2., 3., 4., 5., 6., 7., 8., 9., 10., 11., 12., 13., 14. or 15. group of the periodic table or is a cation from the lanthanides or is NH₄⁺;
more preferably, CatINORGⁿ⁺ is a cation selected from the 1., 2., 4., 5., 6., 7., 8., 9., 10., 11., 12., 13., 14. or 15. group of the periodic table or is a cation from the lanthanides or NH₄⁺;
even more preferably, CatINORGⁿ⁺ is selected from the group consisting of Li⁺, Na⁺, K⁺, Rb⁺, Cs⁺, Be²⁺, Mg²⁺, Ca²⁺, Sr²⁺, Ba²⁺, Ti⁴⁺, Ti³⁺, Zr⁴⁺, Zr³⁺, Hf⁴⁺, Hf³⁺, V⁴⁺, V³⁺, V²⁺, Nb⁴⁺, Ta⁴⁺, Cr³⁺, Mo⁴⁺, Mo³⁺, Mo²⁺, W⁴⁺, W³⁺, W²⁺, Mn⁴⁺, Mn³⁺, Mn²⁺, Fe⁴⁺, Fe³⁺, Fe²⁺, Ru⁴⁺, Ru³⁺, Ru²⁺, Os⁴⁺, Os³⁺, Os²⁺, Co⁴⁺, Co³⁺, Co²⁺, Rh⁴⁺, Rh³⁺, Ir⁴⁺, Ir³⁺, Ni⁴⁺, Ni³⁺, Ni²⁺, Pd⁴⁺, Pd¹⁺, Pd²⁺, Pt⁴⁺, Pt³⁺, Pt²⁺, Cu⁴⁺, Cu³⁺, Cu²⁺, Cu⁺, Ag⁴⁺, Ag³⁺, Ag²⁺, Ag⁺, Au³⁺, Au²⁺, Au⁺, Zn²⁺, Zn⁺, Cd²⁺, Cd⁺, Hg²⁺, Hg⁺, B³⁺, Al³⁺, Ga³⁺, Ga⁺, In³⁺, In⁺, Tl³⁺, Tl⁺, Ge⁴⁺, Ge²⁺, Sn⁴⁺, Sn²⁺, Pb⁴⁺, Pb²⁺, As³⁺, Sb³⁺, Bi³⁺, Bi¹⁺, La³⁺, Nb³⁺, Sm³⁺, Eu³⁺, Gd³⁺, and NH₄⁺;
especially, CatINORGⁿ⁺ is selected from the group consisting of Li⁺, Na⁺, K⁺, Mg²⁺, Ca²⁺, Ti⁴⁺, Ti³⁺, Zr⁴⁺, Zr³⁺, V⁴⁺, V³⁺, V²⁺, Cr³⁺, Mo⁴⁺, Mo³⁺, Mo²⁺, W⁴⁺, W³⁺, W²⁺, Mn⁴⁺, Mn³⁺, Mn²⁺, Fe⁴⁺, Fe³⁺, Fe²⁺, Ru⁴⁺, Ru³⁺, Ru²⁺, Co⁴⁺, Co³⁺, Co²⁺, Rh⁴⁺, Rh³⁺, Ir⁴⁺, Ir³⁺, Ni⁴⁺, Ni³⁺, Ni²⁺, Pd⁴⁺, Pd³⁺, Pd²⁺, Pt⁴⁺, Pt³⁺, Pt²⁺, Cu⁴⁺, Cu³⁺, Cu²⁺, Cu⁺, Ag⁴⁺, Ag³⁺, Ag²⁺, Ag⁺, Zn²⁺, Zn⁺, Al³⁺, Ga³⁺, Ga⁺, In³⁺, In⁺, Sn⁴⁺,Sn²⁺, Pb⁴⁺, Pb²⁺, Sb³⁺, Nb³⁺, Sm³⁺, Eu³⁺, Gd³⁺, and NH₄⁺;
more especially, CatINORGⁿ⁺ is selected from the group consisting of Li⁺, Na⁺, K⁺, Mg²⁺, Ca²⁺, Ti⁴⁺, V⁴⁺, V³⁺, V²⁺, Cr³⁺, Fe⁴⁺, Fe³⁺, Fe²⁺, Co⁴⁺, Co³⁺, Co²⁺, Cu⁴⁺, Cu³⁺, Cu²⁺, Cu⁺, Ag²⁺, Ag⁺, Zn²⁺, Zn⁺, Al³⁺, Sn⁴⁺,Sn²⁺, Pb⁴⁺, Pb²⁺, Sb³⁺, Eu³⁺, Gd³⁺, and NH₄⁺;
even more especially, CatINORGⁿ⁺ is selected from the group consisting of Li⁺, Na⁺, K⁺, Mg²⁺, Ca²⁺, Ti⁴⁺, V⁴⁺, V³⁺, Cr³⁺, Fe⁴⁺, Fe³⁺, Fe²⁺, Co⁴⁺, Co³⁺, Co²⁺, Cu⁴⁺, Cu³⁺, Cu²⁺, Cu⁺, Ag⁺, Zn²⁺, Al³⁺, Sn⁴⁺,Sn²⁺, Pb⁴⁺, Pb²⁺, Gd³⁺, and NH₄⁺;
in particular, CatINORGⁿ⁺ is selected from the group consisting of Li⁺, Na⁺, K⁺, NH₄⁺, Ag⁺, Mg²⁺, Ca²⁺, Zn²⁺ and Cu²⁺;
more in particular, CatINORGⁿ⁺ is selected from the group consisting of Li⁺, Na⁺, K⁺, NH₄⁺, Ag⁺, Mg²⁺, Ca²⁺ and Zn²⁺;
even more in particular, CatINORGⁿ⁺ is Li⁺, Na⁺, K⁺, Ag⁺, Mg²⁺, or Zn²⁺;
especially in particular, CatINORGⁿ⁺ is Li⁺, K⁺, Ag⁺, Mg²⁺, or Zn²⁺.

Preferably, n in CatInORGⁿ⁺ is 1 or 2.

The term "where applicable" in the definition of CatORGⁿ⁺ means, that any of the optional substituents of the residues R2 to R11 requires a respective site, and e.g. in case of R2 being a perfluorinated side chain no respective site is available any more for a substituent.

Preferably, CatORGⁿ⁺ contains a heteroatom selected from the group consisting of nitrogen, phosphorus, sulfur and oxygen;
more preferably, CatORGⁿ⁺ contains a heteroatom selected from the group consisting of nitrogen and phosphorus.

Preferably,
- R16: is selected from the group consisting of C₂₋₆ alkylen, C₅₋₆ cycloalkylen, phenylen, C(H)(phenyl), R17(-O-R17)ₙ₁;
- R17: is selected from the group consisting of CH₂-CH₂, CH₂-CH₂-CH₂ and CH₂-CH₂-CH₂-CH₂;
- R18 and R19: are identical or different and independently from each other selected from the group consisting of H, C₁₋₄ alkyl, C₅₋₆ cycloalkyl, phenyl and benzyl;
- n1: is an integer from 1 to 10; more preferably,
- R16: is selected from the group consisting of C₂₋₄ alkylen, C₆ cycloalkylen, phenylen, C(H)(phenyl), R17(-O-R17)ₙ₁;
- R17: is selected from the group consisting of CH₂-CH₂ and CH₂-CH₂-CH₂;
- R18 and R19: are identical and selected from the group consisting of H, C₁₋₄ alkyl, C₅₋₆ cycloalkyl, phenyl and benzyl;
- n1: is an integer from 1 to 6;
even more preferably, for n being 2 CatORGⁿ⁺ is (H₂(R18)N-R16-N(R19)H₂)²⁺;
- R16: is selected from the group consisting of C₂₋₄ alkylen, phenylen and C(H)(phenyl);
- R18 and R19: are identical and selected from the group consisting of H, C₁₋₄ alkyl, C₅₋₆ cycloalkyl, phenyl and benzyl;
especially, when n is 2, then CatORGⁿ⁺ is (H₃N-CH₂-CH₂-NH₃)²⁺.

Preferably, n in CatORGⁿ⁺ is 1.

Preferably, CatORGⁿ⁺ is selected from the group consisting of ammonium, phosphonium, sulfonium, pyrrolidinium, pyrrolinium, pyrrolium, pyrazolium, pyrazolinium, imidazolium, imidazolinium, triazolium, oxazolium, thiazolium, piperidinium, piperazinium, morpholinium, pyridinium, pyridazinium, pyrimidinium, pyrazinium, 1,3-dioxolium, pyrylium, thiopyrylium, quinoxalinium, indolinium, indolium, [(CH₃)₃SiFSi(CH₃)₃]⁺, Ph₃C⁺, and mixtures thereof;
more preferably from the group consisting of ammonium, phosphonium, sulfonium, pyrrolidinium, pyrrolinium, pyrrolium, pyrazolium, imidazolium, triazolium, oxazolium, thiazolium, piperidinium, piperazinium, morpholinium, pyridinium, pyridazinium, pyrimidinium, pyrazinium, 1,3-dioxolium, pyrylium, thiopyrylium, [(CH₃)₃SiFSi(CH₃)₃]⁺, Ph₃C⁺, and mixtures thereof.

More preferably, CatORGⁿ⁺ is selected from the group consisting of 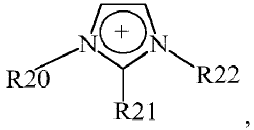 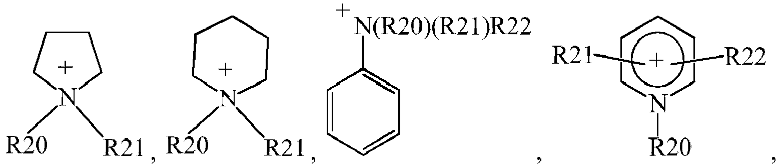 [N(R20)(R21)(R22)R23]⁺, [P(R20)(R21)(R22)R23]⁺, [(CH₃)₃SiFSi(CH₃)₃]⁺, Ph₃C⁺, and mixtures thereof;
wherein
- R20, R21, R23: are identical or different and independently from each other selected from the group consisting of H, C₁₋₂₀ alkyl, C₃₋₁₀ cycloalkyl and allyl;
- R22: is C₁₋₂₀ alkyl, C₃₋₁₀ cycloalkyl or allyl;
preferably,
- R20, R21, R23: are identical or different and independently from each other selected from the group consisting of H, C₁₋₁₄ alkyl, C₅₋₈ cycloalkyl and allyl;
- R22: is C₁₋₁₄ alkyl, C₅₋₈ cycloalkyl or allyl;
more preferably,
- R20, R21, R23: are identical or different and independently from each other selected from the group consisting of H, C₁₋₈ alkyl, C₅₋₇ cycloalkyl and allyl;
- R22: is C₁₋₈ alkyl, C₅₋₇ cycloalkyl or allyl;
even more preferably, CatORGⁿ⁺ is selected from the group consisting of 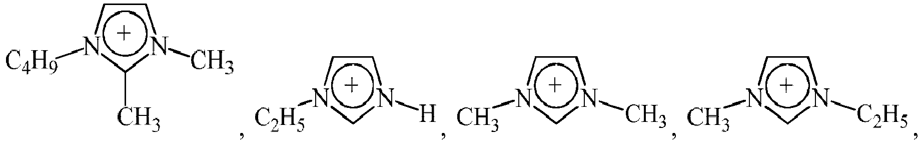 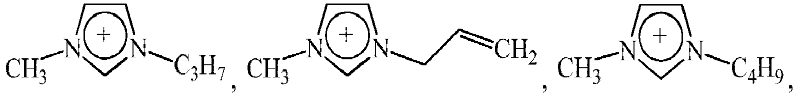 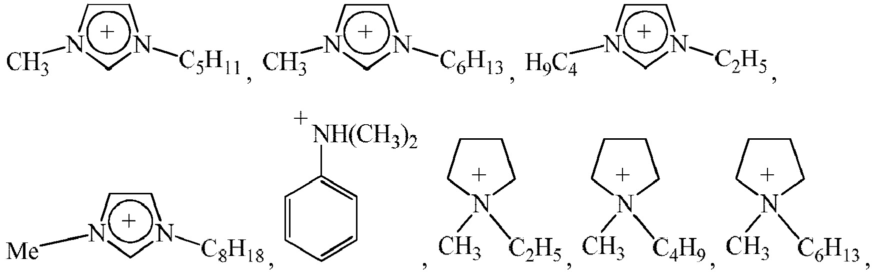 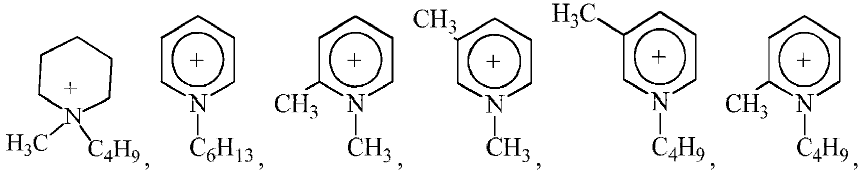 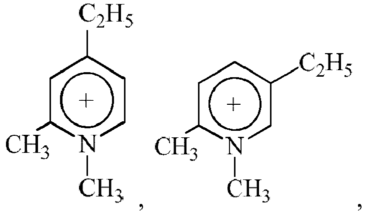 [NH(C₂H₅)₃]⁺, [NH(C₃H₇)₃]⁺, [NH(C₄H₉)₃]⁺, [N(C₂H₅)₄]⁺, [N(C₃H₇)₄]⁺, [N(C₄H₉)₄]⁺, [P(C₂H₅)₄]⁺, [P(C₃H₇)₄]⁺, [P(C₄H₉)₄]⁺, [P(C₆H₁₃)₃(C₁₄H₂₉)]⁺, [(CH₃)₃SiFSi(CH₃)₃]⁺, Ph₃C⁺, and mixtures thereof;
especially, CatORGⁿ⁺ is selected from the group consisting of 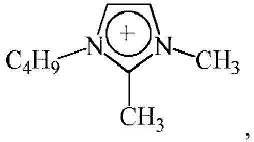 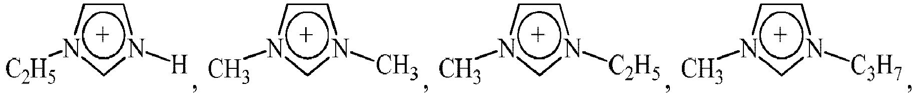 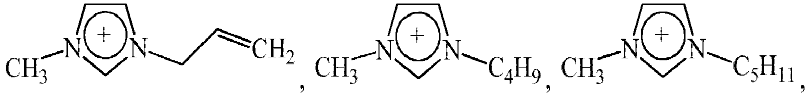 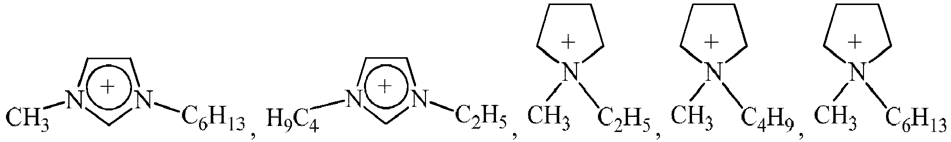 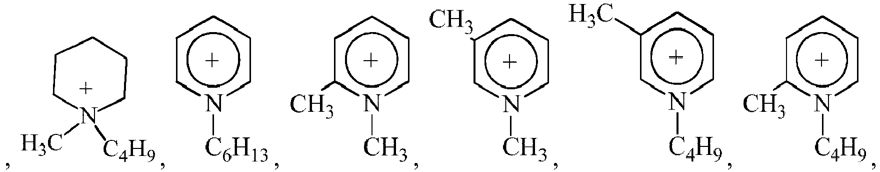 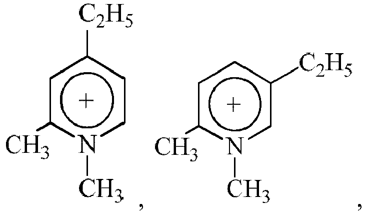 [NH(C₂H₅)₃]⁺, [NH(C₃H₇)₃]⁺, [NH(C₄H₉)₃]⁺, [N(C₂H₅)₄]⁺, [N(C₃H₇)₄]⁺, [N(C₄H₉)₄]⁺, [P(C₂H₅)₄]⁺, [P(C₃H₇)₄]⁺, [P(C₄H₉)₄]⁺, [(CH₃)₃SiFSi(CH₃)₃]⁺, Ph₃C⁺, and mixtures thereof.
more especially, CatORGⁿ⁺ is selected from the group consisting of 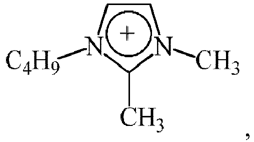 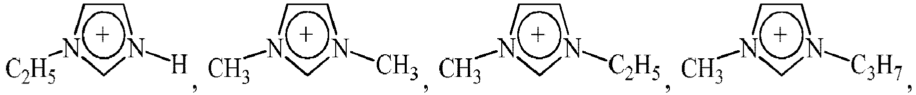 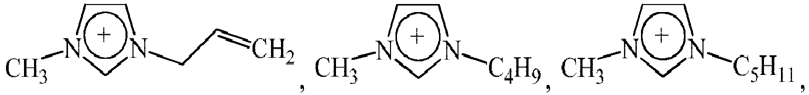 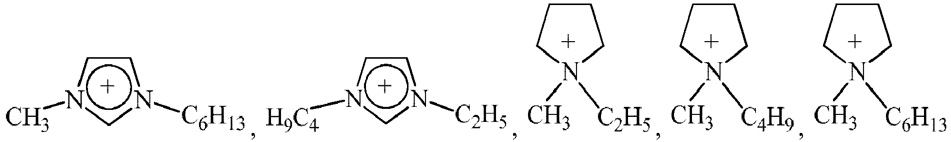 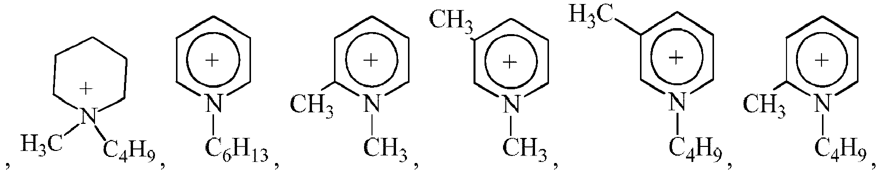 [NH(C₂H₅)₃]⁺, [NH(C₃H₇)₃]⁺, [NH(C₄H₉)₃]⁺, [N(C₂H₅)₄]⁺, [N(C₃H₇)₄]⁺, [N(C₄H₉)₄]⁺, [P(C₂H₅)₄]⁺, [P(C₃H₇)₄]⁺, [P(C₄H₉)₄]⁺, [(CH₃)₃SiFSi(CH₃)₃]⁺, Ph₃C⁺, and mixtures thereof.

In particular, Catⁿ⁺ is a cation (Cat-Part1);
cation (Cat-Part1) is CatINORGⁿ⁺ or CatORGⁿ⁺,
with CatINORGⁿ⁺ selected from the group consisting of Li⁺, Na⁺, K⁺, NH₄⁺, Ag⁺, Mg²⁺, Ca²⁺ and Zn²⁺;
and
with CatORGⁿ⁺ selected from the group consisting of 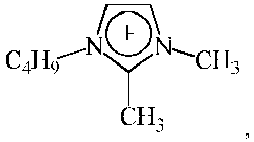 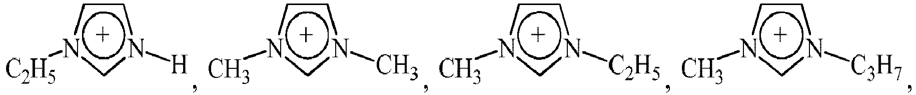 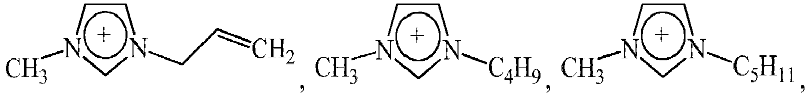 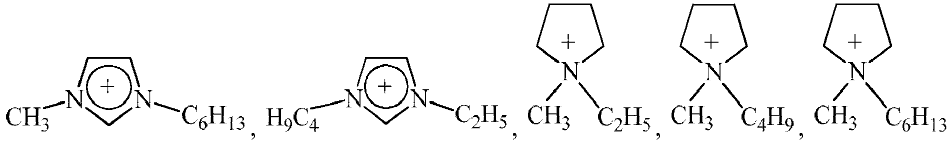 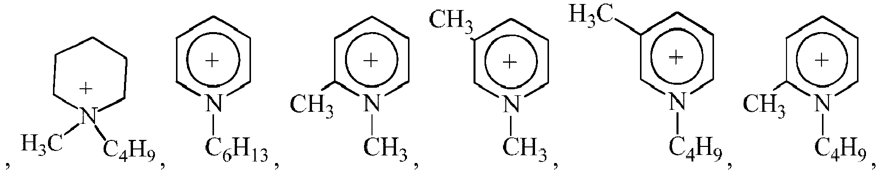 [NH(C₂H₅)₃]⁺, [NH(C₃H₇)₃]⁺, [NH(C₄H₉)₃]⁺, [N(C₂H₅)₄]⁺, [N(C₃H₇)₄]⁺, [N(C₄H₉)₄]⁺, [P(C₂H₅)₄]⁺, [P(C₃H₇)₄]⁺, [P(C₄H₉)₄]⁺, [(CH₃)₃SiFSi(CH₃)₃]⁺, Ph₃C⁺, and mixtures thereof.

Even more preferably, compound of formula (I) is compound (Group-I),
compound (Group-I) is selected from the group consisting of compound of formula (Ia) and compound of formula (Ib);

[Catⁿ⁺][(cis-PF₂(CN)₄)⁻]ₙ (Ia)

[Catⁿ⁺][(mer-PF₃(CN)₃)⁻]ₙ (Ib)

Catⁿ⁺ and n are as defined above, also with all their embodiments, preferably Catⁿ⁺ is cation (Cat-Part1).

In particular, compound of formula (I) is selected from the group consisting of compound of formula (1), compound of formula (2), compound of formula (2a), compound of formula (2b), compound of formula (3), compound of formula (4), compound of formula (4a), compound of formula (4b), and mixtures thereof.

[(n-Bu)₄N][PF₄(CN)₂ (1)

[(n-Bu)₄N][PF₃(CN)₃] (2)

[(n-Bu)₄N][fac-PF₃(CN)₃] (2a)

[(n-Bu)₄N][mer-PF₃(CN)₃] (2b)

[(n-Pr)₃NH][PF₄(CN)₂] (3)

[(n-Pr)₃NH][PF₃(CN)₃] (4)

[(n-Pr)₃NH][fac-PF₃(CN)₃] (4a)

[(n-Pr)₃NH][mer-PF₃(CN)₃] (4b)

Preferably, from 1 to 40 mol equivalents, more preferably 4 to 35 mol equivalents, even more preferably from 5 to 25 mol equivalents, especially from 5 to 15 mol equivalents, of trimethylsilylcyanide are used in REAC1, the mol equivalents being based on the molar amount of the anion [(Z¹F₆)⁻] of compound of formula (A1).

The reaction temperatures of REAC1 is preferably from -75 to 150°C, more preferably from-50 to 120°C, more preferably from -50 to 100°C, even more preferably -50 to 80°C.

Another possible range of the reaction temperatures of REAC1 is preferably from -10 to 150°C, more preferably from -10 to 120°C, more preferably from 0 to 100°C, even more preferably 10 to 80°C.

REAC1 can be done in a closed system and at the pressure caused by the chosen temperature.

The reaction time of REAC1 is preferably from 15 min to 96 h, more preferably from 20 min to 84 h, even more preferably from 20 min to 72 h, especially from 20 min to 60 h.

Preferably, REAC1 is done under inert atmosphere. Preferably, the inert atmosphere is achieved by the use if an inert gas preferably selected from the group consisting of argon, another noble gas, lower boiling alkane, nitrogen and mixtures thereof. The lower boiling alkane is preferably a C₁₋₃ alkane, i.e. methane, ethane or propane.

After the reaction, compound of formula (I) can be isolated by standard methods such as evaporation of volatile components, extraction, washing, drying, concentration, crystallization, chromatography and any combination thereof, which are known per se to the person skilled in the art.

Preferably, after the reaction the reaction product is treated with hydrogen peroxide, preferably with aqueous hydrogen peroxide.

More preferably for isolation, the reaction product is mixed with aqueous hydrogen peroxide to provide a mixture MIXT.

Preferably, the concentration of the aqueous hydrogen peroxide is from 10 to 40 wt% hydrogen peroxide, the wt% based on the total weight of the aqueous hydrogen peroxide.

Preferably, from 1 to 30 mol equivalents, more preferably from 1 to 20 mol equivalents, of hydrogen peroxide are used, the mol equivalents being based on the molar amount of compound of formula (A1).

Preferably, MIXT is stirred for 5 min to 24 h, more preferably for 10 min to 20 h.

Preferably, MIXT is stirred at a temperature TEMPMIXT, TEMPMIXT is preferably from ambient temperature to 100°C.

After treatment with hydrogen peroxide, MIXT is preferably filtrated. The residue of the filtration is preferably washed with a solvent WASHMIXT, WASHMIXT is preferably water or an ether such as diethylether or dichloromethane.

REAC1 can be done in the presence of a compound CAT;
CAT is a Lewis Acid selected from the group consisting of Lewis Acids derived from, that is based on, the 1., 2., 3., 4., 5., 6., 7., 8., 9., 10., 11., 12., 13., 14., 15. and 16. group of the periodic table, zeolite, guanidinium[ANIO] and mixtures thereof;
more preferably, CAT is selected from the group consisting of [(CH₃)₃SiFSi(CH₃)₃][ANIO], Q1(R27)₃, guanidinium[ANIO], (R26)₃C[ANIO], adamantyl[ANIO], [(R24)₃O][ANIO], [(R25)₃Si][ANIO], Q2(R36)(R28)₃, Q3(R29)₃, Q4(R30)₅, Q5(R32)₃, Q6(R33)₂, Q7(R31), Q8(R34)₂, Q9(R35)₃, Q10(R37)₂, Q11(R38), zeolite and mixtures thereof;
even more preferably, CAT is selected from the group consisting of [(CH₃)₃SiFSi(CH₃)₃][ANIO], Si(Cl)(C₆H₅)₃, B(R27)₃, Al(R27)₃, GaF₃, GaCl₃, guanidinium[ANIO], (R26)₃C[ANIO], [(R24)₃O][ANIO], [(R25)₃Si][ANIO], Si(R28)₄, TiF₄, TiCl₄, Q3(halogen)₃, Q3(CN)₃, Q3(C₁₋₄ alkyl)₃, Q4(halogen)₅, Q4(C₁₋₁₀ alkyl)₅, Cr(Cl)₃, Fe(halogen)₃, Mn(Cl)₂, Fe(halogen)₂, Pd(halogen)₂, Pt(halogen)₂, Pd(CN)₂, Pt(CN)₂, Pd(SCN)₂, Pt(SCN)₂, AgCl, AgCN, CuCl, CuCl₂, CuF, CuBr, CuCN, CuF₂, CuBr₂, Cu(CN)₂, ZnF₂, ZnCl₂, ZnBr₂, Zn(CN)₂, ScF₃, ScCl₃, ScBr₃, LnF₃, LnCl₃, LnBr₃, CaCl₂, KF, zeolite and mixtures thereof;
especially, CAT is selected from the group consisting of [(CH₃)₃SiFSi(CH₃)₃][ANIO], Si(Cl)(C₆H₅)₃, B(R27)₃, Al(R27)₃, GaF₃, GaCl₃, (R26)₃C[ANIO], [(R24)₃O][ANIO], [(R25)₃Si][ANIO], Si(halogen)₄, Si(C₁₋₁₀ alkyl)₄, TiF₄, TiCl₄, P(halogen)₃, P(CN)₃, Sb(halogen)₃, Bi(halogen)₃, Bi(CN)₃, P(halogen)₅, P(C₁₋₁₀ alkyl)₅, Sb(halogen)₅, Nb(halogen)₅, CrCl₃, FeF₃, FeCl₃, FeBr₃, MnCl₂, FeF₂, FeCl₂, FeBr₂, PdF₂, PdCl₂, PdBr₂, PtF₂, PtCl₂, PtBr₂, AgCN, CuCl, CuCl₂, CuF, CuBr, CuCN, CuF₂, ZnF₂, ZnCl₂, ZnBr₂, Zn(CN)₂, ScF₃, ScCl₃, LnF₃, LnCl₃, CaCl₂, KF, zeolite and mixtures thereof;
more especially, CAT is selected from the group consisting of [(CH₃)₃SiFSi(CH₃)₃][ANIO], Si(Cl)(C₆H₅)₃, BF₃, BCl₃, BBr₃, B(C₁₋₄ alkyl)₃, B(C₆F₅)₃, AlF₃, AlCl₃, Al(C₁₋₄ alkyl)₃, Al(C₆F₅)₃, GaF₃, GaCl₃, (Ph)₃C[ANIO], (CH₃)₃C[ANIO], [(C₁₋₃ alkyl)₃O][ANIO], [(C₁₋₄ alkyl)₃Si][ANIO], Si(halogen)₄, Si(C₁₋₁₀ alkyl)₄, TiF₄, TiCl₄, P(halogen)₃, P(CN)₃, SbF₃, SbI₃, BiF₃, BiI₃, Bi(CN)₃, P(halogen)₅, SbF₅, NbF₅, NbCl₅, CrCl₃, FeCl₃, FeBr₃, MnCl₂, FeCl₂, FeBr₂, PdCl₂, PdBr₂, PtCl₂, PtBr₂, AgCN, CuCl, CuCl₂, CuF, CuF₂, ZnF₂, ZnCl₂, ZnBr₂, Zn(CN)₂, ScF₃, ScCl₃, LnF₃, LnCl₃, CaCl₂, KF, zeolite and mixtures thereof;
even more especially, CAT is selected from the group consisting of [(CH₃)₃SiFSi(CH₃)₃][ANIO], Si(Cl)(C₆H₅)₃, BF₃, BCl₃, B(C₁₋₄ alkyl)₃, B(C₆F₅)₃, AlCl₃, GaF₃, GaCl₃, (Ph)₃C[ANIO], (CH₃)₃C[ANIO], [(C₁₋₄ alkyl)₃Si][ANIO], SiF₄, SiCl₄, Si(C₁₋₈ alkyl)₄, TiF₄, TiCl₄, PCl₃, PBr₃, PI₃, P(CN)₃, SbF₃, SbI₃, Bi(CN)₃, PF₅, PCl₅, PBr₅, PI₅, SbF₅, NbCl₅, CrCl₃, FeCl₃, FeBr₃, MnCl₂, FeCl₂, FeBr₂, PdCl₂, PdBr₂, PtCl₂, PtBr₂, AgCN, CuCl, CuCl₂, CuF, CuF₂, ZnF₂, Zn(CN)₂, ScF₃, ScCl₃, LnF₃, LnCl₃, CaCl₂, KF, zeolite and mixtures thereof;
in particular, CAT is selected from the group consisting of [(CH₃)₃SiFSi(CH₃)₃][ANIO], Si(Cl)(C₆H₅)₃, BF₃, BCl₃, B(C₆F₅)₃, AlCl₃, GaF₃, GaCl₃, Ph₃C[ANIO], [(C₁₋₄ alkyl)₃Si][ANIO], SiF₄, SiCl₄, Si(C₁₋₄ alkyl)₄, TiF₄, TiCl₄, PCl₃, PBr₃, PI₃, P(CN)₃, SbF₃, SbI₃, Bi(CN)₃, PF₅, PCl₅, PBr₅, PI₅, SbF₅, NbCl₅, CrCl₃, FeCl₃, FeBr₃, MnCl₂, FeCl₂, FeBr₂, PdCl₂, PdBr₂, PtCl₂, PtBr₂, AgCN, CuCl, CuCl₂, CuF, CuF₂, ZnF₂, ScF₃, ScCl₃, LnF₃, LnCl₃, CaCl₂, KF, zeolite and mixtures thereof;
more in particular, CAT is selected from the group consisting of [(CH₃)₃SiFSi(CH₃)₃][ANIO], Si(Cl)(C₆H₅)₃, BF₃, BCl₃, B(C₆F₅)₃, AlCl₃, GaF₃, GaCl₃, Ph₃C[ANIO], SiCl₄, TiF₄, TiCl₄, P(CN)₃, SbF₃, Bi(CN)₃, PF₅, PCl₅, SbF₅, NbCl₅, CrCl₃, FeCl₃, MnCl₂, AgCN, CuCl, CuCl₂, ZnF₂, CaCl₂, KF, zeolite and mixtures thereof;
even more in particular, CAT is selected from the group consisting of [(CH₃)₃SiFSi(CH₃)₃][ANIO], Si(Cl)(C₆H₅)₃, BF₃, B(C₆F₅)₃ GaF₃, GaCl₃, Ph₃C[ANIO], SiCl₄, TiF₄, TiCl₄, P(CN)₃, PF₅, PCl₅, SbF₅, NbCl₅, CrCl₃, FeCl₃, MnCl₂, AgCN, CaCl₂, KF, SiCl₄, zeolite and mixtures thereof;
very even more in particular, CAT is selected from the group consisting of [(CH₃)₃SiFSi(CH₃)₃][ANIO], Si(Cl)(C₆H₅)₃, BF₃, B(C₆F₅)₃ GaF₃, GaCl₃, Ph₃C[ANIO], SiCl₄, TiF₄, TiCl₄, P(CN)₃, PF₅, PCl₅, SbF₅, NbCl₅, CrCl₃, FeCl₃, MnCl₂, SiCl₄, zeolite and mixtures thereof;
in a very preferred embodiment, CAT is selected from the group consisting of BF₃, B(C₆F₅)₃ GaF₃, GaCl₃, TiF₄, TiCl₄, PF₅, PCl₅, SbF₅, [(CH₃)₃SiFSi(CH₃)₃] [ANIO], Ph₃C[ANIO], SbF₅, zeolite and mixtures thereof;
in a more very preferred embodiment, CAT is [(CH₃)₃SiFSi(CH₃)₃][ANIO], B(C₆F₅)₃ GaF₃, GaCl₃, TiF₄, TiCl₄, PF₅, PCl₅, SbF₅, Ph₃C[ANIO], zeolite or mixtures thereof;
in an even more very preferred embodiment, CAT is [(CH₃)₃SiFSi(CH₃)₃][ANIO], B(C₆F₅)₃ GaF₃, GaCl₃, TiF₄, TiCl₄, PF₅, PCl₅, SbF₅, Ph₃C[ANIO], zeolite or mixtures thereof;
in an especially very preferred embodiment, CAT is B(C₆F₅)₃ GaF₃, GaCl₃, TiF₄, TiCl₄, PF₅, PCl₅, SbF₅, Ph₃C[ANIO], zeolite or mixtures thereof;
- Q1: is selected from the group consisting of B, Al and Ga;
- R27: is selected from the group consisting of C₁₋₁₀ alkoxy, halogen, C₁₋₁₀ alkyl, CN, SCN and C₆F₅;

- R24: is C₁₋₁₀ alkyl;
- R25: is C₁₋₁₀ alkyl;
- R26: is selected from the group consisting of CN, SCN, Ph and C₁₋₁₀ alkyl;
- Q2: is selected from the group consisting of Si and Ti;
- R28 and R36: are identical or different and independently from each other selected from the group consisting of C₁₋₁₀ alkoxy, halogen, C₁₋₁₀ alkyl, CN, SCN and C₆F₅;
- Q3: is selected from the group consisting of P, Sb and Bi;
- R29: is selected from the group consisting of C₁₋₁₀ alkoxy, halogen, CN, SCN, C₁₋₁₀ alkyl and C₆F₅;
- Q4: is selected from the group consisting of P, Sb and Nb;
- R30: is selected from the group consisting of C₁₋₁₀ alkoxy, halogen, CN, SCN, C₁₋₁₀ alkyl and C₆F₅;
- Q5: is selected from the group consisting of Cr and Fe;
- R32: is selected from the group consisting of halogen, CN and SCN;
- Q6: is selected from the group consisting of Mn, Fe, Pd and Pt;
- R33: is selected from the group consisting of halogen, CN and SCN;
- Q7: is Cu or Ag;
- R31: is selected from the group consisting of halogen, CN and SCN;
- Q8: is selected from the group consisting of Cu, Zn, Cd and Hg;
- R34: is selected from the group consisting of halogen, CN, and SCN;
- Q9: Sc or Ln;
- R35: is selected from the group consisting of halogen, CN, and SCN;
- Q10: Ca;
- R37: is halogen;
- Q11: K;
- R38: is halogen;
- ANIO: is selected from the group consisting of [P(R40)₆₋ₘ₁(R41)ₘ₁]⁻, [B(R42)₄₋ₘ₂(R43)ₘ₂]⁻, F⁻, Cl⁻, Br⁻, I⁻, CN⁻ and SCN⁻;
- R40 and R41: are identical of different in independently from each other selected from the group consisting of CN, SCN, F, Cl, Br and I;
- m1: is 0, 1, 2, 3, 4 or 5;
- R42 and R43: are identical of different in independently from each other selected from the group consisting of C₆F₅, CN, SCN, F, Cl, Br and I;
- m2: is 0, 1, 2 or 3;
preferably, ANIO is selected from the group consisting of P(R40)₆⁻, B(R42)₄⁻, F⁻, Cl⁻, Br⁻, I⁻, CN⁻ and SCN⁻;
R40 is selected from the group consisting of CN, SCN, F, Cl, Br and I;
R42 is selected from the group consisting of C₆F₅, CN, SCN, F, Cl, Br and I;
more preferably, ANIO is selected from the group consisting of P(R40)₆⁻, B(R42)₄⁻, F , Cl⁻, Br⁻, CN⁻ and SCN⁻;
R40 is selected from the group consisting of CN, SCN, F, Cl and Br;
R42 is selected from the group consisting of C₆F₅, CN, SCN, F, Cl and Br.

Preferably,
Q1 is B.

Preferably,
R24 is C₁₋₄ alkyl;
R25 is C₁₋₇ alkyl;
R26 is selected from the group consisting of Ph and C₁₋₄ alkyl;
R27 is selected from the group consisting of C₁₋₇ alkoxy, Cl, F, Br, C₁₋₇ alkyl and C₆F₅; more preferably,
R24 is C₁₋₃ alkyl;
R25 is C₁₋₅ alkyl;
R26 is selected from the group consisting of Ph and C₁₋₂ alkyl;
R27 is selected from the group consisting of C₁₋₄ alkoxy, Cl, F, C₁₋₄ alkyl and C₆F₅; even more preferably,
R24 is methyl or ethyl;
R25 is C₁₋₄ alkyl;
R26 is Ph or methyl;
R27 is selected from the group consisting of C₁₋₃ alkoxy, Cl, F, C₁₋₃ alkyl and C₆F₅.

Preferably, [ANIO] is [B(C₆F₅)₄] or [PF₆].

In a special embodiments, CAT is selected from the group consisting of [(CH₃)₃SiFSi(CH₃)₃][B(C₆F₅)₄], Si(Cl)(C₆H₅)₃, BF₃, BCl₃, B(C₆F₅)₃, AlCl₃, GaF₃, GaCl₃, Ph₃C[PF₆], SiCl₄, TiF₄, TiCl₄, P(CN)₃, SbF₃, Bi(CN)₃, PF₅, PCl₅, SbF₅, NbCl₅, CrCl₃, FeCl₃, MnCl₂, AgCN, CuCl, CuCl₂, ZnF₂, CaCl₂, KF, zeolite, and mixtures thereof;
preferably, CAT is selected from the group consisting of [(CH₃)₃SiFSi(CH₃)₃][B(C₆F₅)₄], Si(Cl)(C₆H₅)₃, BF₃, B(C₆F₅)₃, GaF₃, GaCl₃, Ph₃C[PF₆], SiCl₄, TiF₄, TiCl₄, P(CN)₃, PF₅, PCl₅, SbF₅, NbCl₅, CrCl₃, FeCl₃, MnCl₂, SiCl₄, zeolite, and mixtures thereof;
more preferably, CAT is selected from the group consisting of [(CH₃)₃SiFSi(CH₃)₃][B(C₆F₅)₄], BF₃, B(C₆F₅)₃ GaF₃, GaCl₃, Ph₃C[PF₆], TiF₄, TiCl₄, PF₅, PCl₅, SbF₅, zeolite, and mixtures thereof;
even more preferably, CAT is [(CH₃)₃SiFSi(CH₃)₃][B(C₆F₅)₄], B(C₆F₅)₃ GaF₃, GaCl₃, TiF₄, TiCl₄, PF₅, PCl₅, SbF₅, Ph₃C[PF_{6]}, zeolite or mixtures thereof;
especially, CAT is B(C₆F₅)₃, GaF₃, GaCl₃, TiF₄, TiCl₄, PF₅, PCl₅, SbF₅, Ph₃C[PF₆], zeolite or mixtures thereof.

In another preferred embodiment, CAT is selected from the group consisting of (CH₃)₃SiFSi(CH₃)₃[B(C₆F₅)₄], [Ph₃C][PF₆], B(C₆F₅)₃ and mixtures thereof;
more preferably, CAT is [Ph₃C][PF₆].

CAT can be used in immobilized form on a carrier CARR;
CARR is a carrier conventionally used for immobilizing catalysts in heterogeneously catalyzed reactions;
preferably, CARR is seleceted from the group consisting of epoxide, polystyrene, zeolite, activated carbon and metal oxide;
said metal oxide is preferably selected from the group consisting of MnO₂, Fe₂O₃, Co₃O₄, NiO, CuO, CuMnO₂, MgO, Al₂O₃, SiO₂, V₂O₅, MoO₃, WO₃ and mixed oxides thereof.

Zeolite can be any zeolite, preferably montmorrilonte or bentonite, more preferably Montmorillonite K10®, BASF, Germany (also available at Sigma Aldrich, CAS Number 1318-93-0).

Compound of formula (A1) and CAT can be one and the same compound, therefore in one preferred embodiment, compound of formula (A1) is different from CAT; in another preferred embodiment, compound of formula (A1) is identical with CAT.

Preferably, from 0.0001 to 40 mol equivalents, more preferably 0.001 to 35 mol equivalents, even more preferably from 0.005 to 25 mol equivalents, especially from 0.005 to 25 mol equivalents, more especially from 0.005 to 15 mol equivalents, even more especially from 0.005 to 5 mol equivalents, of CAT are used in REAC1, the mol equivalents being based on the combined molar amount of the anion [(Z¹F₆)⁻] of compound of formula (A1), CATACID and CAT.

In another preferable embodiment, from 0.01 to 40 mol%, more preferably 0.1 to 35 mol%, even more preferably 0.1 to 25 mol%, especially from 0.5 to 15 mol%, more especially from 0.5 to 10 mol%, even more especially from 0.5 to 5 mol%, of CAT are used in REAC1, the mol% being based on the combined molar amount of the anion [(Z¹F₆)⁻] of compound of formula (A1), CATACID and CAT.

Preferably, from 0.01 to 40 mol%, more preferably 0.1 to 35 mol%, even more preferably 0.1 to 25 mol%, especially from 0.5 to 20 mol%, more especially from 0.5 to 10 mol%, even more especially from 0.5 to 5 mol%, of CATACID are used in REAC1, the mol% being based on the combined molar amount of the anion [(Z¹F₆)⁻] of compound of formula (A1), CATACID and, if present, CAT;

Preferably, the method comprises additionally to STEP1 a step STEP2, STEP2 is done after STEP1;
STEP2 comprises a reaction REAC2, REAC2 is a metathesis reaction wherein cation Catⁿ⁺ in compound of formula (1) is exchanged for a cation different from Catⁿ⁺;
compound of formula (I) having been prepared in STEP1;
Catⁿ⁺, n, compound of formula (I) and STEP1 are as defined above, also with all their embodiments.

REAC2 is a metathesis reaction, also called a salt-exchange reaction. In a metathesis reaction such as REAC2 a first cation in a first salt is exchanged for a second cation, said second cation coming from a second salt.

Preferably, REAC2 provides for the preparation of a compound of formula (I-Cat-r);

[Cat-r^{r+}] [(Z¹F₆₋ₘ(CN)ₘ)⁻]r (I-Cat-r)

- Cat-r^{r+}: is selected from the group consisting of CatINORGⁿ⁺ and CatORGⁿ⁺ and is different from Catⁿ⁺;
- r: is 1, 2, 3 or 4;
with STEP1, Z¹, m, CatINORGⁿ⁺ and CatORGⁿ⁺ as defined above, also with all their embodiments.

Preferably, in REAC2 Catⁿ⁺ is exchanged for Cat-r^{r+} from a compound of formula (I-Cat-n);

(Cat-r^{r+})ₜ₁ (AnINORG^{q-})ₜ₂ (I-Cat-n)

- q: is 1 or 2;
- t1: is 1 or2;
- t2: is 1, 2, 3 or 4;
when r is 1 and q is 1, then t1 is 1 and t2 is 1;
when r is 2 and q is 1, then t1 is 1 and t2 is 2;
when r is 3 and q is 1, then t1 is 1 and t2 is 3;
when r is 4 and q is 1, then t1 is 1 and t2 is 4;
when r is 1 and q is 2, then t1 is 2 and t2 is 1;
when r is 2 and q is 2, then t1 is 1 and t2 is 1;
when r is 3 and q is 2, then t1 is 2 and t2 is 3;
when r is 4 and q is 2, then t1 is 1 and t2 is 2;
AnINORG^{q-} is an anion selected from the group consisting of halide, OH⁻, CN⁻, OCN , SCN⁻, N₃⁻, sulfate, hydrogensulfate, nitrate, CO₃²⁻, HCO₃⁻, BF₄⁻, PF₆⁻, SbF₆⁻, CF₃SO₃⁻, (CF₃SO₂)₂N⁻, (FSO₂)₂N⁻, C₁₋₆ alkyl-SO₃⁻, C₁₋₆ alkyl-O-SO₃⁻, 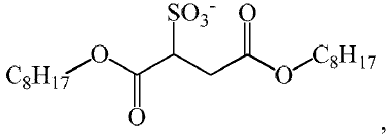 anions of C₁₋₂₀ monocarboxylic aliphatic acids, mono- and dianions of C₂₋₆ dicarboxylic aliphatic acids, anions of benzoic acids, mono-and dianions of phthalic acids, of isophthalic acids and of terephthalic acids, N(CN)₂⁻, C(CN)₃⁻, B(CN)₄⁻, P(CN)₆⁻, Sb(CN)₆⁻, and mixtures thereof;
Cat-r^{r+}, r, CatINORGⁿ⁺ and CatORGⁿ⁺ are as defined above, also with all their embodiments.

Preferably, AnINORG^{q-} is an anion selected from the group consisting of halide, OH⁻, CN⁻, sulfate, hydrogensulfate, nitrate, CO₃²⁻, HCO₃⁻, BF₄⁻, PF₆⁻, CF₃SO₃⁻, (CF₃SO₂)₂N⁻, (FSO₂)₂N⁻, H₃C-SO₃⁻, H₃C-CH₂-SO₃⁻, H₃C-O-SO₃⁻, H₃C-CH₂-O-SO₃⁻, acetate, oleate, fumarate, maleate, oxalate, benzoate, N(CN)₂⁻, and mixtures thereof;
more preferably, AnINORG^{q-} is an anion selected from the group consisting of Br , Cl⁻, OH⁻, CN , sulfate, hydrogensulfate, CO₃²⁻, HCO₃⁻, acetate, and mixtures thereof;
even more preferably, AnINORG^{q-} is an anion selected from the group consisting of Cl⁻, OH⁻, CN⁻, sulfate, hydrogensulfate, CO₃²⁻, HCO₃⁻, acetate, and mixtures thereof.

In another preferred embodiment, AnINORG^{q-} is an anion selected from the group consisting of halide, OH⁻, CN⁻, OCN⁻, SCN⁻, N₃⁻, sulfate, hydrogensulfate, nitrate, CO₃²⁻, HCO₃⁻, BF₄⁻, PF₆⁻, SbF₆⁻, CF₃SO₃⁻, (CF₃SO₂)₂N⁻, (FSO₂)₂N⁻, C₁₋₆ alkyl-SO₃⁻, C₁₋₆ alkyl-O-SO₃⁻, 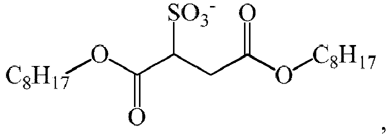 anions of C₁₋₂₀ monocarboxylic aliphatic acids, anions of C₂₋₆ dicarboxylic aliphatic acids, benzoate, phthalates, N(CN)₂⁻, C(CN)₃⁻, B(CN)₄⁻, P(CN)₆⁻, Sb(CN)₆⁻, and mixtures thereof.

Preferably, r is 1 or 2.

In case of REAC2, preferably a compound of formula (I-Cat-r) with Cat-r^{r+} being CatORGⁿ⁺ is prepared by exchange of a Catⁿ⁺ being a CatINORGⁿ⁺ in compound of formula (I) for a CatORGⁿ⁺.

Said CatORGⁿ⁺ is provided in REAC2 preferably in form of a compound of formula (I-CatORG)

(CatORGⁿ⁺)_{q}(AnINORG^{q-})ₙ (I-CatORG)

wherein
Catⁿ⁺, n, CatORGⁿ⁺, CatINORGⁿ⁺, q and AnINORG^{q-} are as defined above, also with all their embodiments.

Preferably, in REAC2 the cation different from Catⁿ⁺, that is preferably Cat-r^{r+}, is present in at least such a molar amount relative to the molar amount of Catⁿ⁺ as required for a stoichiometric exchange of said two cations;
more preferably, compound of formula (I) and compound of formula (I-Cat-n) are present in at least such a molar amount relative to each other, that Catⁿ⁺ is stoichiometrically exchanged for Cat-r^{r+};
even more preferably, the molar amount of compound of formula (I-Cat-n) is such, that from 1 to 1.5, even more preferably from 1 to 1.2, required equivalents of Cat-r^{r+} relative to the equivalents of Catⁿ⁺ are present.

The reaction temperatures of REAC2 is preferably from 0 to 250 °C, more preferably from 10 to 200 °C, even more preferably from 10 to 150 °C, especially from 10 to 100°C, more especially from 10 to 50°C.

The REAC2 is preferably carried out in a solvent SOLV2, SOLV2 is preferably selected from the group consisting of water, DCM, ethyl acetate, C₅₋₁₀ alkane, and mixtures thereof. C₅₋₁₀ alkane is preferably pentane, hexane or heptane.

In a more preferred embodiment, REAC2 is done in DCM or in a biphasic solvent system of water and DCM.

As an alternative, REAC2 can also be carried out in the absence of a solvent or in a solvent in which the inorganic salt formed as side product is sparingly soluble or insoluble. As a further alternative, it is also possible to carry out the reaction in an aqueous solution using an ion exchanger loaded with the desired cation Catⁿ⁺.

The amount of solvent is preferably from 2 to 40 fold, more preferably from 3 to 20 fold, of the weight of compound of formula (I).

REAC2 can be done in a closed system and at the pressure caused by the chosen temperature.

The reaction time of REAC2 is preferably from 15 min to 96 h, more preferably from 15 min to 48 h, even more preferably from 15 min to 24 h.

Preferably, REAC2 is done under inert atmosphere. Preferably, the inert atmosphere is achieved by the use if an inert gas preferably selected from the group consisting of argon, another noble gas, lower boiling alkane, nitrogen and mixtures thereof. The lower boiling alkane is preferably a C₁₋₃ alkane, i.e. methane, ethane or propane.

Subsequent to REAC2 there can be a further metathesis reaction or further metathesis reactions.

After REAC2, compound of formula (I) can be isolated from the reaction mixture by standard methods such as filtration, evaporation of volatile components, extraction, washing, drying, concentration, crystallization, chromatography and any combination thereof, which are known per se to the person skilled in the art.

For example, when REAC2 was done in a biphasic solvent system of water and DCM, the aqueous and organic phases are separated, the organic phase is preferably washed, preferably with water, then preferably dried, preferably with Na₂SO₄, K₂CO₃, CaCl₂ or MgSO₄, and finally evaporated.

Or as another example, when REAC2 was done in DCM and a suspension was formed, filtration and evaporation of the solvent will isolate the product.

It is possible to use compound of formula (I), which was obtained by the method of instant invention, as substrate in a similar reaction with trimethylsilylcyanide.

Therefore the method of instant invention can comprise additionally to STEP1 a step STEP1-1, STEP1-1 is done after STEP1;
STEP1-1 comprises a reaction REAC1-1, wherein compound of formula (I), obtained in STEP1, is reacted with trimethylsilylcyanide;
preferably, REAC1-1 is done in the presence of CATACID, in the presence of CAT, or in the presence of both CATACID and CAT;
with CAT and CATACID as defined above, also in all their embodiments.

CATACID is commercially available compounds or can be prepared according to known methods. Compounds of formula (A1) are commercially available depending on the cation Catⁿ⁺, e.g. [(n-Bu₄)N][PF₆], [(n-Pr)₄N][PF₆], K[PF₆] and Li[PF₆], as well as CAT are commercially available. Other compounds of formula (A1) with cations Catⁿ⁺ different from K⁺ and (n-Bu₄)N⁺, and which are not commercially available, can be prepared by conventional metathesis reaction, i.e. substitution of the respective cation K⁺ or (n-Bu₄)N⁺ against another cation, or by reaction of hexafluorophosphoric acid with a respective compound, such as exemplified herein.

### EXAMPLES

### Methods

¹H, ¹³C, ¹⁹F and ³¹P NMR spectra were recorded on a Bruker AVANCE 300 and Bruker AVANCE 250 instruments in CD₃CN, CDCl₃, D₆-DMSO, D₂O or CD₂Cl₂. Chemical shifts are expressed in parts per million referred to TMS in case of ¹H and ¹³C, C¹⁹FCl₃ in case of ¹⁹F, and H₃³¹PO₄ in case of ³¹P, and coupling constants (J) in Hertz. When a % value for the amount of compounds is stated based on NMR measurement, the % value represents an area-%, the area-% being based on the total area of peaks in the spectrum. In case of the individual amount of a component in a mixture the stated % value for the amount of the component in the mixture represents an area-%, this area-% being based on the combined area of peaks of all components of the mixture; if not stated otherwise.

IR-spectra were recorded on a Nicolet 380 FT-IR spectrometer. Measurements were done at ambient temperature.

RAMAN-spectra were recorded on a LabRAM HR 800 Horiba Jobin YVON. Measurements were done at ambient temperature.

The C/H/N-analyses were measured on a C/H/N/S-Analysator (Thermoquest Flash EA 1112).

Melting points and temperature of decomposition T_{dec} were measured on a DSC 823e from Mettler-Toledo. The calibration was carried out with the melting points of In (156.6 ± 0.3°C) and Zn (419.6 ± 0.7°C) with an heating rate of 5 K per min.

TGA/DSC measurements were conducted on a Setaram Labsys TGA / DSC 1600. The measurements were carried out under argon atmosphere with a heating rate of 5 K per min, corrected via a blank measurement.

### Preparation description A: Synthesis of [(n-Bu)₄N][PF₆]

A solution of [(n-Bu₄)N]Br (41.91 g, 130 mmol) in 200 ml of CH₂Cl₂ was added to a solution of K[PF₆] (23.93 g, 130 mmol) in 200 ml of H₂O. After stirring for 24 h at ambient temperature the phases were separated. The organic phase was washed three times with 30 ml of water, dried over anhydrous Na₂CO₃ and filtered. The filtrate was concentrated on a rotary evaporator and [(n-Bu₄)N][PF_{6]} as a white solid was obtained. The product was recrystallized from hot ethanol and dried at 80°C in vacuo for 10 hours to yield 47.3 g (94%, 122 mmol).
**DSC** (10 K/min): m.p. = 250°C, Tdec = 388°C
**C/H/N Analysis** calc. % (found): C 49.60 (49.76), H 9.37 (9.28), N 3.61 (3.50)
**¹H NMR** (25°C, CD₃CN, 300.13 MHz, delta in ppm): 0.96 (t, 12H, CH₃), 1.34 (m, 8H, CH₃-CH₂), 1.59 (m, 8H, CH₂-CH₂N), 3.06 (m, 8H, NCH₂)
**¹⁹F NMR** (25°C, CD₃CN, 300.13 MHz, delta in ppm): -73.0 (d, 6F, PF₆, ¹J(³¹P-¹⁹F) = 707 Hz)
**³¹P NMR** (25°C, CD₃CN, 300.13 MHz, delta in ppm): -144.6 (sept, 1P, PF₆, ¹J(³¹P-¹⁹F) = 707 Hz)
**IR** (ATR, 32 scans, in cm⁻1): 2966 (m), 2937 (w), 2879 (w), 1472 (m), 1404 (w), 1386 (w), 1360 (w), 1350 (w), 1319 (w), 1260 (w), 1242 (w), 1165 (w), 1109 (w), 1070 (w), 1035 (w), 931 (w), 880 (m), 829 (s), 738 (m), 555 (s)

### Preparation description B: Synthesis of [(n-Pr)₃NH][PF₆]

Hexafluorophosphoric acid (10 mL, 55 wt% aqueous solution, 113 mmol) was mixed with destilled water (50 ml) and cooled to ca. 0 °C. n-Pr₃N (21.6 ml, 113 mmol) was added dropwise to the mixture. After 15 min of stirring the resulting white solid was filtered and washed with water. The white solid was recrystallized from a hot mixture of water : ethanol (1:3). After drying in vacuo 30 g (92 %, 104 mmol) of [(n-Pr)₃NH][PF₆] were obtained.
**DSC** (10 K/min): **T_{dec}** = 260 °C
**C/H/N Analysis** % calc. (found): C 37.37 (37.35) H 7.67 (7.62) N 4.84 (4.74)
**¹H NMR** (300 K, CD₂Cl₂, 300.13 MHz, delta in ppm): 1.00 (t, 9H, CH₃), 1.75 (m, 6H., CH₂-CH₃), 3.09 (m, 6H, NCH₂), 6.11 (br, 1H, NH)
**¹³C{¹H} NMR** (300 K, CD₂Cl₂, 75.47 MHz, delta in ppm): 10.9 (3C, s, CH₃), 17.8 (s, 3C, CH₂-CH₃), 56.1 (s, 3C, NCH₂)
**¹⁹F NMR** (300 K, CD₂Cl₂, 282.40 MHz, delta in ppm): -71.9 (d, 6F, ¹J(¹⁹F-³¹P) = 712 Hz)
**³¹P NMR** (300 K, CD₂Cl₂, 121.49 MHz, delta in ppm): -144.3 (sept, 1P, ¹J(³¹P-¹⁹F) = 713 Hz)
**IR** (25 °C, ATR, 64 scans, cm⁻¹): 555 (s), 741 (m), 756 (s), 818 (s), 878 (m), 970 (w), 987 (w), 1045 (w), 1095 (w), 1244 (w), 1269 (w), 1414 (w), 1462 (w), 1475 (w), 2885 (w), 2945 (w) 2978 (w), 3221 (w)
**Raman** (25°C, 12 mW, 5 scans, cm⁻¹): 312 (3), 469 (1), 741 (10), 822 (1), 920 (1), 1040 (4), 1091 (1), 1108 (1), 1314 (1), 1332 (1), 1456 (4), 2748 (1), 2885 (5), 2943 (6), 2982 (5)

### Example 1: Synthesis of compound of formula (2)

[(n-Bu)₄N][PF₆] (811 mg, 2.09 mmol), prepared according to Preparation Description A, triflic acid (9 mol%, the mol% being based on the combined molar amount of [(n-Bu)₄N][PF₆] and triflic acid, 30 mg, 0.2 mmol) and (CH₃)₃SiCN (2.08 g, 21 mmol) were stirred under argon atmosphere at ambient temperatures. After 2 hours of stirring a ¹⁹F NMR and ³¹P spectra were measured. According to ¹⁹F NMR and ³¹P NMR spectrum the reaction mixture was a mixture of 50 % of compound of formula (1) and 50 % of compound of formula (2). After 53 hours of stirring another ¹⁹F NMR spectrum was measured. In accordance with the ¹⁹F NMR the product contained more than 99 % of compound of formula (2) with around 97 % of compound of formula (2b) and 3 % of compound of formula (2a).
**¹⁹F NMR** (300 K, CD₃CN, 282.40 MHz, delta in ppm): -54.4 (dt, 2F, cis-PF₄(CN)₂, ¹J(¹⁹F-³¹P) = 764 Hz), -41.6 (d, 3F, fac-PF₃(CN)₃, ¹J(¹⁹F-³¹P) = 739 Hz), -41.3 (dt, 2F, cis-PF₄(CN)₂, ¹J(¹⁹F-³¹P) = 712 Hz), -40.1 (dd, 2F, mer-PF₃(CN)₃, ¹J(¹⁹F-³¹P) = 678 Hz), -31.4 (d, 4F, trans-PF₄(CN)₂, ¹J(¹⁹F-³¹P) = 740 Hz), -9.9 (dt, 1F, mer-PF₃(CN)₃, ¹J(¹⁴⁹F-³¹P) = 777 Hz)
**³¹P NMR** (300 K, CD₃CN, 121.49 MHz, delta in ppm): -170.8 (quin, 1P, trans-PF₄(CN)₂, ¹J(³¹P-¹⁹F) = 738 Hz), -183.3 (tt, 1P, cis-PF₄(CN)₂, ¹J(³¹P-¹⁹F) = 765 Hz, ¹J(³¹P-¹⁹F) = 713), -207.5 (dt, 1P, mer-PF₂(CN)₃, ¹J(³¹P-¹⁹F) = 685 Hz, ¹J(³¹P-¹⁹F) = 780 Hz)

### Example 2: Synthesis of compound of formula (4)

[(n-Pr)₃NH][PF₆] (2.66 g, 9.20 mmol), prepared according to Preparation Description B, triflic acid (17 mol%, the mol% being based on the combined molar amount of [(n-Pr)₃NH][PF₆] and triflic acid, 205 mg, 1.95 mmol) and (CH₃)₃SiCN (9.13 g, 92 mmol) were stirred under argon atmosphere at ambient temperatures for 2 h. The excess (CH₃)₃SiCN and any (CH₃)₃SiF were removed in vacuo resulting in a light brown oil, which was suspended in aqueous H₂O₂ (7 ml, 70 mmol, 30 wt%) and 10 ml dest. water. After stirring the suspension at ambient temperature for 15 h the suspension was extract two times with 30 ml dichloromethane. The separated organic phases were dried over anhydrous Na₂SO₄ and filtered. The filtrate was concentrated at a rotary evaporator and dried in vacuo for 15 hours to obtain 2.40 g (84 %, 7.73 mmol) of compound of formula (4) as an light orange oil.

According to ¹⁹F and ³¹P NMR the product contained less than 1% impurities of compound of formula (3). The product was a mixture of 96 % of compound of formula (4b) and 4 % of compound of formula (4a).
**DSC** (10 K/min): **T_{dec}** = 210°C
**C/H/N Analysis** % calc. (found.): C 46.45 (46.22) H 7.15 (7.12) N 18.06 (17.37)
**¹H NMR** (300 K, CD₂Cl₂, 250.13 MHz, delta in ppm): 1.06 (t, 9H, CH₃), 1.78 (m, 6H., CH₂-CH₃), 3.07 (m, 6H, NCH₂), 6.80 (br, 1H, NH)
**¹³C{¹H} NMR** (300 K, CD₂Cl₂, 62.90 MHz, delta in ppm): 11.0 (3C, s, CH₃), 17.9 (s, 3C, CH₂-CH₃), 55.9 (s, 3C, NCH₂), 125.6 (ddt, 2C, mer-PF₃(CN)₃, ¹J(¹³C-³¹P) = 260 Hz, ²J(¹³C-¹⁹F) = 80 Hz, ²J(¹³C-¹⁹F) = 53 Hz), 129.2 (ddt, 1C, mer-PF₃(CN)₃, ¹J(¹³C-³¹P) = 180 Hz, ²J(¹³C-¹⁹F) = 35 Hz, ²J(¹³C-¹⁹F) = 19 Hz)
**¹⁹F NMR** (300 K, CD₃CN, 282.40 MHz, delta in ppm):-41.6 (d, 3F, fac-PF₃(CN)₃, ¹J(¹⁹F-³¹P) = 751 Hz, -39.7 (dd, 2F, mer-PF₃(CN)₃, ¹J(¹⁹F-³¹P) = 690 Hz, -10.0 (dt, 1F, mer-PF₃(CN)₃, ¹J(¹⁹F-³¹P) = 788 Hz
**³¹P NMR** (300 K, CD₂Cl₂, 101.25 MHz, delta in ppm): -224.2 (quar, 1P, fac-PF₃(CN)₃, ¹J(³¹P-¹⁹F) = 740 Hz), -209.9 (dt, 1P, mer-PF₂(CN)₃, ¹J(³¹P-¹⁹F) = 684 Hz, ¹J(³¹P-¹⁹F) = 779 Hz, ¹J(³¹P-¹³C) = 260, ¹J(³¹P-¹³C) = 180)
**IR** (25 °C, ATR, 32 scans, cm⁻¹): 552 (s), 596 (m), 640 (m), 683 (s), 754 (m), 793 (s), 829 (s), 982 (w), 1041 (w), 1093 (w), 1171 (w), 1240 (w), 1389 (w), 1427 (w), 1462 (w), 1473 (m), 2199 (w), 2208 (w), 2806 (w), 2885 (w), 2945 (w), 2976 (w), 3093 (w)

### Comparative Example 1 - no CATACID

[(n-Bu)₄N][PF₆] (1.00 g, 2.58 mmol), prepared according to Preparation Description A, and (CH₃)₃SiCN (2.58 g, 26 mmol) were stirred under argon atmosphere at ambient temperature for 100 h.

After stirring at ambient temperature for 100 h a ¹⁹F NMR spectrum was measured.

In accordance to ¹⁹F NMR the product contained about 1% of compound of formula (5) and 99% of [(n-Bu)₄N][PF₆].

[(n-Bu)₄N][PF₅(CN)] (5)

**¹⁹F NMR** (25°C, d6-DMSO, 300.13 MHz, delta in ppm): -77.7 (dquin, 1F, PF₅(CN), ¹J(³¹P-¹⁹F) = 760 Hz), -73.4 (d, 6F, PF₆, ¹J(³¹P-¹⁹F) = 710 Hz), -49.4 (d, 4F, PF₅(CN), ¹J(³¹P-¹⁹F) = 741 Hz)

## Claims

1. Method for the preparation of compound of formula (I);
[Catⁿ⁺] [(Z¹F₆₋ₘ(CN)ₘ)⁻]ₙ (I)
the method comprises a step STEP1;
STEP1 comprises a reaction REAC1, wherein compound of formula (A1)
[Catⁿ⁺] [(Z¹F₆)⁻]ₙ (A1)
is reacted with trimethylsilylcyanide in the presence of a compound CATACID;
CATACID is selected from the group consisting of HF, HCl, HBr, HI, HNO₃, H₂SO₃, H₂SO₄, NaHSO₄, Oleum, KHSO₄, NaHCO₃, KHCO₃, H₃PO₄, H₃PO₃, HSO₃F, HSO₃Cl, CH₃COOH, CF₃COOH, C(Cl₃)COOH, methansulfonic acid, HCOOH, AR-COOH, AR-SO₃H, C(OH)(COOH)₃, CF₃SO₃H HCN, HOCN, HSCN, HSeCN, HNCO, HNCS, HN₃, HQ20(X1)4, polymolybdate, polytungstate, HQ21(X1)₆, and mixtures thereof;
AR is Phe or Phe substituted with 1, 2 or 3 identical or different residues selected from the group consisting of halogen and CH₃;
Q20 is B, Al, Ga, In or Th;
Q21 is P, As, Sb or Bi;
X1 is halogen;
Z¹ is selected from the group consisting of P, As, Sb and Bi;
m is 1, 2, 3, 4 or 5;
n is 1, 2, 3 or 4;
Catⁿ⁺ is selected from the group consisting of inorganic cation CatINORGⁿ⁺ and organic cation CatORGⁿ⁺;
CatINORGⁿ⁺ is a cation selected from the 1., 2., 3., 4., 5., 6., 7., 8., 9., 10., 11., 12., 13., 14., 15. or 16. group of the periodic table, or is a cation from the lanthanides or is a cation from the actinides or is NH₄⁺;
CatORGⁿ⁺ is selected from the group consisting of CatORG-A⁺, CatORG-B⁺, CatORG-C⁺, [(CH₃)₃SiFSi(CH₃)₃]⁺, Ph₃C⁺, guanidinium and (H₂(R18)N-R16-N(R19)H₂)²⁺;
CatORG-A⁺ is (WR2R3R4R5)⁺,
wherein
W is a nitrogen or phosphorus; and
(i) R2, R3, R4 and R5 are identical or different and independently from each other selected from the group consisting of H, C₁₋₂₀ alkyl, C₁₋₂₀ perfluoroalkyl, C₃₋₁₀ cycloalkyl and C₆₋₁₀ aryl, with the proviso, that at least one of the residues R2, R3, R4 and R5 is not H; or
(ii) R2 and R3 together are a hydrocarbon chain and form together with W a 5- to 7-membered saturated or unsaturated heterocyclic ring,
R4 and R5 are identical or different and independently from each other selected from the group consisting of H, C₁₋₂₀ alkyl, C₁₋₂₀ perfluoroalkyl, C₃₋₁₀ cycloalkyl and C₆₋₁₀ aryl; or
(iii) R2 and R3 together are a hydrocarbon chain and form together with W, and R4 and R5 together are a hydrocarbon chain and form together with W, independently from each other, 5- to 7-membered saturated or unsaturated heterocyclic rings;
CatORG-B⁺ is (XR6R7R8)⁺,
wherein
X is nitrogen,
R6 and R7 together are a hydrocarbon chain and form together with X a 5- to 7-membered unsaturated heterocyclic ring in which X is connected by a single bond and a double bond to R6 and R7 respectively,
R8 is selected from the group consisting of H, C₁₋₂₀ alkyl, C₂₋₈ alkenyl, C₁₋₂₀ perfluoroalkyl, C₃₋₁₀ cycloalkyl or C₆₋₁₀ aryl;
CatORG-C⁺ is (YR9R10R11)⁺,
wherein
Y is sulphur;
(i) R9, R10 and R11 are identical or different and independently from each other selected from the group consisting of H, C₁₋₂₀ alkyl, C₁₋₂₀ perfluoroalkyl, C₃₋₁₀ cycloalkyl and C₆₋₁₀ aryl; or
(ii) R9 and R10 together are a hydrocarbon chain and form together with Y a 5- to 7-membered saturated or unsaturated ring,
R11 is selected from the group consisting of H, C₁₋₂₀ alkyl, C₁₋₂₀ perfluoroalkyl, C₃₋₁₀ cycloalkyl and C₆₋₁₀ aryl;
the residues R2, R3, R4, R5, R6, R7, R8, R9, R10 and R11 are, independently from each other, unsubstituted or, where applicable, substituted by 1, 2, 3, 4, 5 or 6 substituents selected from the group consisting of C₁₋₄ alkyl, C₃₋₁₀ cycloalkyl, C₂₋₈ alkenyl, phenyl, benzyl, halogen, cyano and C₁₋₄ alkoxy;
in any of said hydrocarbon chains formed by R2 and R3, by R4 and R5, by R6 and R7, and by R9 and RI0 1 or 2 carbon atoms of said hydrocarbon chains can be exchanged for 1 or 2 heteroatoms respectively, said one or two heteroatoms being selected from the group consisting of O, N and S; in case of an exchange for N, this N is unsubstituted or substituted by a residue selected from the group consisting of C₁₋₈ alkyl, C₃₋₁₀ cycloalkyl, C₂₋₈ alkenyl and C₁₋₈ perfluoroalkyl;
R16 is selected from the group consisting of C₂₋₈ alkylen, C₃₋₈ cycloalkylen, phenylen, C(H)(phenyl), R17(-O-R17)ₙ₁;
R17 is selected from the group consisting of CH₂-CH₂, CH₂-CH₂-CH₂, CH₂-C(H)(CH₃)-CH₂, CH₂-CH₂-C(H)(CH₃) and CH₂-CH₂-CH₂-CH₂;
R18 and R19 are identical or different and independently from each other selected from the group consisting of H, C₁₋₈ alkyl, C₃₋₈ cycloalkyl, phenyl and benzyl;
n1 is an integer from 1 to 20.

2. Method according to claim 1, wherein
Z¹ is P.

3. Method according to claim 1 or 2, wherein
m is 2, 3, 4 or 5.

4. Method according to one or more of claims 1 to 3, wherein
n is 1 or 2.

5. Method according to one or more of claims 1 to 4, wherein
CATACID is selected from the group consisting of HF, HCl, HBr, H₂SO₄, NaHSO₄, Oleum, KHSO₄, H₃PO₄, HSO₃F, HSO₃Cl, CH₃COOH, CF₃COOH, C(Cl₃)COOH, methansulfonic acid, HCOOH, AR-SO₃H, C(OH)(COOH)₃, CF₃SO₃H, HCN, HQ20(X1)4, polymolybdate, polytungstate, HQ21(X1)₆, and mixtures thereof;
AR is Phe or Phe substituted with 1, 2 or 3 identical or different residues selected from the group consisting of halogen and CH₃;
Q20 is B, Al or Ga;
Q21 is P, Sb or Bi;
X1 is F, Cl or Br.

6. Method according to one or more of claims 1 to 5, wherein
CatINORGⁿ⁺ is a cation selected from the 1., 2., 3., 4., 5., 6., 7., 8., 9., 10., 11., 12., 13., 14. or 15. group of the periodic table or is a cation from the lanthanides or is NH₄⁺.

7. Method according to one or more of claims 1 to 6, wherein
CatINORGⁿ⁺ is a cation selected from the 1., 2., 4., 5., 6., 7., 8., 9., 10., 11., 12., 13., 14. or 15. group of the periodic table or is a cation from the lanthanides or NH₄⁺.

8. Method according to one or more of claims 1 to 7, wherein
CatINORGⁿ⁺ is selected from the group consisting of Li⁺, Na⁺, K⁺, Rb⁺, Cs⁺, Be²⁺, Mg²⁺, Ca²⁺, Sr²⁺, Ba²⁺, Ti⁴⁺, Ti³⁺, Zr⁴⁺, Zr³⁺, Hf⁴⁺, Hf³⁺, V⁴⁺, V³⁺, V²⁺, Nb⁴⁺, Ta⁴⁺, Cr³⁺, Mo⁴⁺, Mo³⁺, Mo²⁺, W⁴⁺, W³⁺, W²⁺, Mn⁴⁺, Mn³⁺, Mn²⁺, Fe⁴⁺, Fe³⁺, Fe²⁺, Ru⁴⁺, Ru³⁺, Ru²⁺, Os⁴⁺, Os³⁺, Os²⁺, Co⁴⁺, Co³⁺, Co²⁺, Rh⁴⁺, Rh³⁺, Ir⁴⁺, Ir³⁺, Ni⁴⁺, Ni³⁺, Ni²⁺, Pd⁴⁺, Pd³⁺, Pd²⁺, Pt⁴⁺, Pt³⁺, Pt²⁺, Cu⁴⁺, Cu³⁺, Cu²⁺, Cu⁺, Ag⁴⁺, Ag³⁺, Ag²⁺, Ag⁺, Au³⁺, Au²⁺, Au⁺, Zn²⁺, Zn⁺, Cd²⁺, Cd⁺, Hg²⁺, Hg⁺, B³⁺, Al³⁺, Ga³⁺, Ga⁺, In³⁺, In⁺, Tl³⁺, Tl⁺, Ge⁴⁺, Ge²⁺, Sn⁴⁺, Sn²⁺, Pb⁴⁺, Pb²⁺, As³⁺, Sb³⁺, Bi³⁺, Bi¹⁺, La³⁺, Nb³⁺, Sm³⁺, Eu³⁺, Gd³⁺, and NN₄⁺.

9. Method according to one or more of claims 1 to 8, wherein
CatORGⁿ⁺ is selected from the group consisting of ammonium, phosphonium, sulfonium, pyrrolidinium, pyrrolinium, pyrrolium, pyrazolium, pyrazolinium, imidazolium, imidazolinium, triazolium, oxazolium, thiazolium, piperidinium, piperazinium, morpholinium, pyridinium, pyridazinium, pyrimidinium, pyrazinium, 1,3-dioxolium, pyrylium, thiopyrylium, quinoxalinium, indolinium, indolium, [(CH₃)₃SiFSi(CH₃)₃]⁺, Ph₃C⁺, and mixtures thereof.

10. Method according to one or more of claims 1 to 9, wherein CatORGⁿ⁺ is selected from the group consisting of 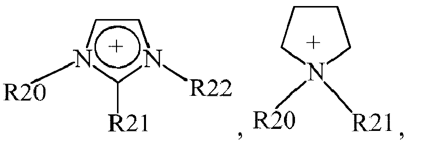 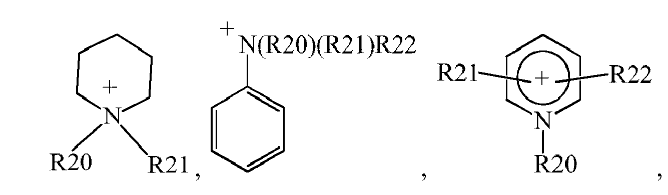 [N(R20)(R21)(R22)R23]⁺, [P(R20)(R21)(R22)R23]⁺, [(CH₃)₃SiFSi(CH₃)₃]⁺, Ph₃C⁺, and mixtures thereof;
wherein
R20, R21, R23 are identical or different and independently from each other selected from the group consisting of H, C₁₋₂₀ alkyl, C₃₋₁₀ cycloalkyl and allyl;
R22 is C₁₋₂₀ alkyl, C₃₋₁₀ cycloalkyl or allyl.

11. Method according to one or more of claims 1 to 10, wherein
compound of formula (I) is compound (Group-I),
compound (Group-I) is selected from the group consisting of compound of formula (Ia) and compound of formula (Ib).
[Catⁿ⁺][(cis-PF₂(CN)₄)⁻]ₙ (Ia)
[Catⁿ⁺] [(mer-PF₃(CN)₃)⁻]ₙ (Ib)

12. Method according to one or more of claims 1 to 11, wherein
compound of formula (I) is selected from the group consisting of compound of formula (1), compound of formula (2), compound of formula (2a), compound of formula (2b), compound of formula (3), compound of formula (4), compound of formula (4a), compound of formula (4b), and mixtures thereof.
[(n-Bu)₄N][PF₄(CN)_{2]} (1)
[(n-Bu)₄N][PF₃(CN)₃] (2)
[(n-Bu)₄N][fac-PF₃(CN)₃] (2a)
[(n-Bu)₄N][mer-PF₃(CN)₃] (2b)
[(n-Pr)₃NH][PF₄(CN)₂] (3)
[(n-Pr)₃NH][PF₃(CN)₃] (4)
[(n-Pr)₃NH][fac-PF₃(CN)₃] (4a)
[(n-Pr)₃NH][mer-PF₃(CN)₃] (4b)

13. Method according to one or more of claims 1 to 12, wherein
REAC1 is done in the presence of a compound CAT;
CAT is a Lewis Acid selected from the group consisting of Lewis Acids derived from, that is based on, the 1., 2., 3., 4., 5., 6., 7., 8., 9., 10., 11., 12., 13., 14., 15. and 16. group of the periodic table, zeolite, guanidinium[ANIO] and mixtures thereof.

14. Method according to claim 13, wherein
CAT is selected from the group consisting of [(CH₃)₃SiFSi(CH₃)₃][ANIO], Q1(R27)₃, guanidinium[ANIO], (R26)₃C[ANIO], adamantyl[ANIO], [(R24)₃O][ANIO], [(R25)₃Si][ANIO], Q2(R36)(R28)₃, Q3(R29)₃, Q4(R30)₅, Q5(R32)₃, Q6(R33)₂, Q7(R31), Q8(R34)₂, Q9(R35)₃, Q10(R37)₂, Q11(R38), zeolite and mixtures thereof;
Q1 is selected from the group consisting of B, A1 and Ga;
R27 is selected from the group consisting of C₁₋₁₀ alkoxy, halogen, C₁₋₁₀ alkyl, CN, SCN and C₆F₅;
R24 is C₁₋₁₀ alkyl;
R25 is C₁₋₁₀ alkyl;
R26 is selected from the group consisting of CN, SCN, Ph and C₁₋₁₀ alkyl;
Q2 is selected from the group consisting of Si and Ti;
R28 and R36 are identical or different and independently from each other selected from the group consisting of C₁₋₁₀ alkoxy, halogen, C₁₋₁₀ alkyl, CN, SCN and C₆F₅;
Q3 is selected from the group consisting of P, Sb and Bi;
R29 is selected from the group consisting of C₁₋₁₀ alkoxy, halogen, CN, SCN, C₁₋₁₀ alkyl and C₆F₅;
Q4 is selected from the group consisting of P, Sb and Nb;
R30 is selected from the group consisting of C₁₋₁₀ alkoxy, halogen, CN, SCN, C₁₋₁₀ alkyl and C₆F₅;
Q5 is selected from the group consisting of Cr and Fe;
R32 is selected from the group consisting of halogen, CN and SCN;
Q6 is selected from the group consisting of Mn, Fe, Pd and Pt;
R33 is selected from the group consisting of halogen, CN and SCN;
Q7 is Cu or Ag;
R31 is selected from the group consisting of halogen, CN and SCN;
Q8 is selected from the group consisting of Cu, Zn, Cd and Hg;
R34 is selected from the group consisting of halogen, CN, and SCN;
Q9 Sc or Ln;
R35 is selected from the group consisting of halogen, CN, and SCN;
Q10 Ca;
R37 is halogen;
Q11 K;
R38 is halogen;
ANIO is selected from the group consisting of [P(R40)₆₋ₘ₁(R41)ₘ₁]⁻, [B(R42)₄₋ₘ₂(R43)ₘ₂]⁻, F⁻, Cl⁻, Br⁻, I⁻, CN⁻ and SCN⁻;
R40 and R41 are identical of different in independently from each other selected from the group consisting of CN, SCN, F, Cl, Br and I;
m1 is 0, 1, 2, 3, 4 or 5;
R42 and R43 are identical of different in independently from each other selected from the group consisting of C₆F₅, CN, SCN, F, Cl, Br and I;
m2 is 0, 1, 2 or 3.

15. Method according to one or more of claims 1 to 14, wherein
the method comprises additionally to STEP1 a step STEP2, STEP2 is done after STEP 1; STEP2 comprises a reaction REAC2, REAC2 is a metathesis reaction wherein cation Catⁿ⁺ in compound of formula (I) is exchanged for a cation different from Catⁿ⁺;
compound of formula (I) having been prepared in STEP 1.
